(19) Europäisches Patentamt
European Patent Office
Office européen des brevets

(11) EP 1 723 949 B1

(12) # FASCICULE DE BREVET EUROPEEN

(45) Date de publication et mention de la délivrance du brevet:
**24.03.2010 Bulletin 2010/12**

(51) Int Cl.:
***A61K 9/51** (2006.01)* ***A61K 8/00** (2006.01)*
***A61Q 19/00** (2006.01)*

(21) Numéro de dépôt: **06300477.4**

(22) Date de dépôt: **16.05.2006**

(54) **Particules d'huile gélifiée pour le ciblage de glandes sébacées et/ou de follicules pileux.**

Partikel aus geliertem Öl, zur gezielten Ansteuerung der Talgdrüsen und der Haarfollikel

Gelified oil particles targeting the sebaceous glands and hair follicles

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **17.05.2005 FR 0551275**

(43) Date de publication de la demande:
**22.11.2006 Bulletin 2006/47**

(73) Titulaire: **L'Oréal**
**75008 Paris (FR)**

(72) Inventeurs:
- **Biatry, Bruno**
**94300 Vincennes (FR)**
- **Simonnet, Jean-Thierry**
**94240 Cachan (FR)**

(74) Mandataire: **Le Coupanec, Pascale A.M.P. et al**
**Nony & Associés,**
**3 rue de Penthièvre**
**75008 Paris (FR)**

(56) Documents cités:
**WO-A-00/61083 WO-A-02/092043**
**FR-A- 2 868 295 US-B1- 6 468 551**

- **RUIZ MARTINEZ MA A ET AL: "Influence of the concentration of a gelling agent and the type of surfactant on the rheological characteristics of oleogels." FARMACO (LAUSANNE), vol. 58, no. 12, décembre 2003 (2003-12), pages 1289-1294, XP002364382 ISSN: 0014-827X**
- **AIACHE J M ET AL: "New gelification method for vegetable oils I: cosmetic application" INTERNATIONAL JOURNAL OF COSMETIC SCIENCE, BLACKWELL SCIENTIFIC PUBLICATIONS, OXFORD, GB, vol. 14, no. 5, 1992, pages 228-234, XP002093985 ISSN: 0142-5463**
- **ROLLAND A ET AL: PHARMACEUTICAL RESEARCH, vol. 10, no. 12, 12 janvier 1993 (1993-01-12), pages 1738-1744, XP008010243**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 723 949 B1

## Description

La présente invention concerne des particules huileuses calibrées et sphériques, notamment adaptées à un ciblage des follicules pileux et/ou des glandes sébacées.

La présente invention concerne également une composition cosmétique ou dermatologique comprenant ces particules, ainsi qu'un procédé de fabrication de ces particules.

Le follicule pileux et la glande sébacée sont, généralement, associés dans une unité dite "pilo-sébacée", bien que certaines régions de la peau peuvent comprendre des glandes sébacées non annexées à des poils. A l'exception de la paume des mains et de la face plantaire des pieds et des orteils, les glandes sébacées sont présentes sur l'ensemble de la peau, y compris les lèvres. Leur densité varie en fonction de la région considérée.

Cette unité, ou follicule, pilo-sébacée est formée d'une invagination tubulaire de l'épiderme, s'enfonçant dans le derme. Les glandes sébacées secrètent un produit lipidique, le sébum, qui peut influer sur le caractère gras ou sec de la peau. L'unité pilo-sébacée peut faire l'objet d'un processus inflammatoire, altérant notamment l'aspect de la peau, et dont les causes peuvent être variées. Par exemple, il peut être provoqué par la présence de microorganismes.

De part sa morphologie, cette unité peut constituer une voie de passage potentielle pour des agents destinés à agir, soit sur des tissus cutanés profonds, par exemple pour les agents de type anti-rides profonds, de type amincissant, soit sur les tissus cutanés superficiels, pour lesquels elle agit comme réservoir de substance, soit, le cas échéant, pour permettre la pénétration de substances actives, jusqu'au niveau de la circulation sanguine et leur diffusion dans l'ensemble de l'organisme.

Différentes méthodes visant précisément à tirer profit de cette voie de passage pour cibler des substances actives au niveau du follicule pileux et/ou des glandes sébacées ont déjà été proposées. La glande sébacée peut être ainsi utilisée en tant que réservoir de ces substances actives qui sont ensuite relarguées pour atteindre leur site d'action, comme par exemple, la peau ou les cheveux.

Ainsi, des particules polymériques présentant un réseau de pores susceptibles de contenir une molécule active destinée à être libérée à partir de la particule ont été proposées (voir US 4,690,825). Ces particules polymériques sont obtenues par polymérisation de polymère de type styrène et divinyle benzène, et présentent un diamètre variant de 10 à 100 microns.

Des capsules comprenant des microparticules imprégnées d'une substance active dispersée dans une phase huileuse de type silicone, éventuellement épaissie ou gélifiée ont également été décrites (voir WO 99/53904). Ces microparticules sont obtenues par polymérisation d'un mélange de monomères monoéthyléniques insaturés ou polyéthyléniques insaturés en présence d'un agent porogène de manière à obtenir un réseau de pores. Ces capsules présentent un diamètre moyen d'environ 1 μm à environ 500 μm.

Toutefois, les microparticules décrites dans les documents évoqués ci-dessus présentent l'inconvénient, soit de nécessiter un solvant pour être chargées en substances actives, soit de nécessiter l'introduction de la substance active lors de l'étape de polymérisation, entraînant ainsi un risque de dégradation de la substance active. Par ailleurs, l'utilisation de ces particules poreuses peut imposer des contraintes de formulation destinées à limiter la fuite des substances actives dans le reste de la composition.

Des particules de nature huileuse ont été proposées à titre d'alternative.

Ainsi, l'usage de particules d'un diamètre d'environ 50 μm à 10 mm et comportant une huile structurée par un agent de gélification pour formuler une composition de nettoyage de la peau et des cheveux a été proposée (voir US 6,737,394).

De WO 02/092043, est connue une composition de soin pour la peau comportant une phase aqueuse dans laquelle est dispersée une phase huileuse structurée à l'aide d'un agent gélifiant. La phase huileuse, dont la viscosité ne dépasse pas 500 Pa.s, y est dispersée sous la forme de particules d'une taille variant de 1 à 500 μm.

De EP 0 375 520, est connue une composition cosmétique pour application topique comportant des particules de corps gras, comprenant un produit actif, et présentant un diamètre compris entre 3 et 10 μm.

Toutefois, l'ensemble des particules précédemment décrites sont obtenues au moyen de procédés ne permettant pas la production de particules d'une part calibrées dans un intervalle de taille prédéterminé et, par ailleurs, compatibles pour un ciblage optimal des follicules pileux et/ou des glandes sébacées.

Or, le calibrage des particules est précisément avantageux dans la mesure où il garantit à celles-ci des distributions de taille et de forme homogènes et une stabilité et des performances, notamment en terme de véhicule pour des substances actives, accrues.

Au sens de la présente invention, on entend désigner par "substances actives", des molécules ou substances susceptibles d'exercer un effet, après application sur une partie du corps humain ou animal.

Il existe par conséquent un besoin pour optimiser le ciblage et la pénétration de substances actives au niveau de la glande sébacée et/ou du follicule pileux.

Il existe également un besoin pour améliorer la stabilité du chargement de la substance active dans des particules susceptibles de pénétrer la glande sébacée et/ou le follicule pileux.

Il existe encore un besoin pour obtenir des particules huileuses, structurées, destinées au ciblage de la glande sébacée et/ou du follicule pileux qui soient stables et qui n'exsudent pas dans le temps.

Il existe encore un besoin pour obtenir des particules huileuses susceptibles de pénétrer dans la glande sébacée et/ou le follicule pileux qui soient homogènes dans leur structure et leur distribution de taille.

Il existe également un besoin pour permettre un relargage des substances actives à partir de la glande sébacée et/ou du follicule pileux selon un cycle de temps optimum.

Il existe encore un besoin d'obtenir des particules huileuses permettant un stockage et une libération progressive de substances actives à partir de la glande sébacée et/ou du follicule pileux, de manière à favoriser le contact prolongé de ces dernières avec leur site d'action, et d'améliorer ainsi leur efficacité.

Il existe également un besoin pour obtenir des particules huileuses dont l'intégrité est maintenue lors de l'application sur un support.

La présente invention a précisément pour objet de satisfaire tout ou partie des besoins en s'affranchissant des inconvénients précédemment évoqués.

Les inventeurs ont observés qu'il était possible d'obtenir à partir d'une phase huileuse structurée par au moins un polymère gélifiant des particules huileuses calibrées et sphériques qui soient conformées de manière à cibler efficacement les glandes sébacées et/ou le follicule pileux, et notamment y favoriser la pénétration de substances actives, et ainsi à augmenter l'efficacité de ces dernières.

La présente invention, selon un des ses premiers aspects a ainsi pour objet des particules huileuses calibrées et sphériques comprenant au moins une phase huileuse structurée par au moins un polymère gélifiant, lesdites particules possédant une taille moyenne inférieure ou égale à 15 $\mu$m, ladite phase huileuse structurée possédant un point de fusion supérieur ou égal à 40 °C et leur indice de circularité étant compris entre 0,9 et 1.

La présente invention à également pour objet des particules huileuses calibrées et sphériques comprenant au moins une phase huileuse structurée par au moins un polymère gélifiant et possédant une taille moyenne inférieure ou égale à 15 $\mu$m, le polymère gélifiant étant choisi parmi un polymère semi-cristallin, un polyamide, un polyamide siliconé, un monoalkylester ou un polyalkylester de polysaccharide à l'exception des esters d'acide gras de dextrine, un copolymère dibloc et/ou tribloc et/ou multibloc et/ou radial-bloc, et leurs mélanges, et leur indice de circularité étant compris entre 0,9 et 1.

La présente invention, selon un autre de ses aspects, a également pour objet des particules huileuses calibrées et sphériques obtenues à partir d'une phase huileuse structurée par au moins un polymère gélifiant, le polymère gélifiant étant de nature et/ou à une teneur suffisante pour conférer à ladite phase huileuse une viscosité supérieure ou égale à 750 Pa.s au cisaillement de 1 $s^{-1}$, à 25 °C, et leur indice de circularité est compris entre 0,9 et 1.

Au sens de la présente invention, on entend désigner par "teneur suffisante", la teneur minimale nécessaire pour observer l'effet attendu, à savoir dans le cas du polymère gélifiant, l'obtention d'une phase huileuse structurée présentant la viscosité requise à l'obtention de particules conformes à la présente invention.

Les particules calibrées et sphériques selon l'invention peuvent comprendre au moins une substance active, qui pourra être, par la conformation des particules, avantageusement ciblée vers les follicules pileux et/ou les glandes sébacées.

Selon encore un autre de ses aspects, la présente invention a pour objet une dispersion en phase aqueuse et/ou hydrosoluble comprenant des particules conformes à la présente invention.

Selon encore un autre de ses aspects, la présente invention a également pour objet un procédé de fabrication d'une dispersion conforme à l'invention.

Selon encore un autre de ses aspects, la présente invention a pour objet une composition cosmétique ou dermatologique comprenant au moins des particules et/ou au moins une dispersion conforme à l'invention.

Selon encore un autre de ses aspects, la présente invention a également pour objet l'utilisation de particules et/ou au moins d'une dispersion conforme à l'invention, dans une composition cosmétique destinée au traitement des peaux grasses.

Selon encore un autre de ses aspects, la présente invention a également pour objet l'utilisation de particules et/ou au moins d'une dispersion conforme à l'invention pour la préparation d'une composition dermatologique destinée au traitement de l'acné.

Selon encore un autre de ses aspects, la présente invention a également pour objet un procédé de maquillage et/ou de soin non thérapeutique de la peau, comprenant au moins une étape d'application sur celle-ci d'au moins une composition conforme à l'invention.

Il est entendu que les compositions conformes à l'invention sont avantageusement destinées à être appliquées sur toute partie de la peau du corps humain, ou animal, comprenant au moins une glande sébacée et/ou au moins un follicule pileux, à savoir toute partie de la peau, y compris les lèvres et le cuir chevelu.

Dans le cadre de la présente invention, les termes « gélifié » et « épaissi » peuvent être considérés comme des synonymes du terme « structuré » lorsqu'il s'agit de qualifier les particules huileuses.

Tous les brevets ou demandes de brevet cités ci-après sont incorporés dans la présente demande par référence.

## PARTICULES CALIBREES ET SPHERIQUES

Les particules huileuses calibrées comprenant au moins une huile ou phase huileuse structurée conformes à l'invention possèdent une taille moyenne inférieure ou égale à 15 μm, en particulier inférieure ou égale à 12 μm, et plus particulièrement inférieure ou égale à 11 μm. Avantageusement, la taille moyenne des particules peut varier de 100 nm à 15 μm, de 100 nm à 12 μm, voire de 150 nm à 11 μm.

On entend par "calibrées", au sens de la présente invention, des particules possédant une distribution granulométrique homogène.

La distribution granulométrique qualifie la répartition de la taille des particules calibrées autour d'une taille moyenne. La distribution granulométrique peut être caractérisée par un indice de polydispersité ou un coefficient d'uniformité. Plus la valeur de l'indice, ou du coefficient, est faible, plus les tailles des particules se répartissent de manière homogène autour d'une taille moyenne.

Ainsi, pour les particules huileuses calibrées submicroniques, conformes à l'invention, c'est-à-dire de taille moyenne inférieure au micromètre, la distribution granulométrique peut être caractérisée par un indice de polydispersité, noté IP (valeur sans dimension et caractérisant l'étendue de la distribution granulométrique). Cet indice est alors avantageusement inférieur ou égal à 0,35, et de préférence supérieur ou égal à 0,01.

La taille des particules huileuses calibrées, submicroniques, peut être déterminée, par exemple, avec un granulomètre laser fonctionnant sur le principe de la diffusion quasi élastique de la lumière, comme le BI90PLUS® de BROOKHAVEN INSTRUMENT.

Pour les particules huileuses calibrées de taille moyenne supérieure au micromètre, la distribution granulométrique peut être caractérisée par un coefficient d'uniformité mesuré à l'aide d'un granulomètre à diffraction laser, comme par exemple le MASTER SIZER 2000® de MALVERN. Les particules huileuses calibrées conformes à l'invention dont la taille est supérieure au micromètre, peuvent posséder un coefficient d'uniformité avantageusement inférieur ou égal à 0,45, et de préférence supérieur ou égal à 0,1.

La taille des particules, et l'homogénéité de distribution granulométrique autour d'une taille moyenne sont généralement déterminées par la nature du procédé utilisé lors de leur obtention. Les procédés permettant d'obtenir les particules calibrées conformes à l'invention sont décrits par la suite.

Les particules calibrées présentent une forme homogène et substantiellement sphérique.

Par "substantiellement sphérique", on entend signifier que les particules sont de forme substantiellement isotrope, c'est-à-dire qu'elles possèdent une morphologie relativement régulière.

On définit ainsi, dans le cadre de la présente invention, un paramètre relatif au facteur de forme des particules comme l'indice de circularité C, défini comme le rapport de la surface totale A de la particule sur la surface du disque ayant le même périmètre P :

$$C = 4\Pi A/P$$

avec C compris entre 0,9 et 1.

Cette mesure peut avantageusement être effectuée avec un appareil Sysmex FPIA 2100, granulomètre par analyse d'images.

Par ailleurs, outre l'homogénéité de forme et de distribution de taille, les particules calibrées et sphériques conformes à l'invention sont avantageusement homogènes quant à leur structure.

Ainsi, le gel huileux constituant la particule et obtenu par la structuration d'au moins une huile ou phase huileuse avec au moins un polymère gélifiant présente avantageusement une structure homogène.

Avantageusement, lorsqu'une substance active est incorporée dans les particules conformes à la présente invention, la distribution de celle-ci dans l'ensemble de la particule est également homogène.

## HUILE

Les particules huileuses calibrées et sphériques conformes à la présente invention comprennent au moins une huile ou phase huileuse, contenant notamment au moins une huile liquide à température ambiante (20 - 25 °C) et à pression atmosphérique.

On entend désigner par "phase huileuse", au sens de la présente invention, une phase comprenant au moins une huile. Cette huile est avantageusement une huile non volatile. Au sens de la présente invention, on entend par "huile non-volatile", une huile ayant une pression de vapeur inférieure à 0,13 Pa.

La phase huileuse, convenant à la mise en oeuvre des particules conformes à la présente invention peut, par exemple, comprendre au moins une huile choisie parmi une huile végétale, une huile animale, une huile synthétique,

minérale, et leurs mélanges.

La phase huileuse peut, également, comprendre en outre au moins une huile volatile qui peut requérir des conditions particulières du procédé, notamment sous pression.

Au sens de la présente invention, on entend par "huile volatile", une huile (ou milieu non aqueux) susceptible de s'évaporer au contact de la peau (par exemple aux environs de 33 °C) en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm Hg), et de préférence allant de 1,3 Pa à 13 000 Pa (0,01 à 100 mm Hg) et préférentiellement allant de 1,3 Pa à 1300 Pa (0,01 à 10 mm Hg).

Les huiles convenant à la mise en oeuvre de l'invention peuvent être d'origine naturelle, végétale ou minérale, ou d'origine synthétique. Elles peuvent être de type hydrocarboné telles que, par exemple, les triglycérides, esters, alcanes, polyoléfines, de type silicone ou de type fluorée, modifiée ou non.

Au sens de la présente invention, on entend par "huile fluorée", une huile comprenant au moins un atome de fluor.

Au sens de la présente invention, on entend par "huile siliconée", une huile comprenant au moins un atome de silicium, et notamment au moins un groupe Si-O.

On entend désigner par "huile hydrocarbonée", une huile contenant principalement des atomes d'hydrogène et de carbone et éventuellement des atomes d'oxygène, d'azote, de soufre et/ou de phosphore.

Selon un mode de réalisation, elles peuvent être utilisées seules ou en mélange, entre elles ou avec d'autres composés tels que définis, par exemple, par la suite.

Avantageusement, les huiles utilisées pour la mise en oeuvre de l'invention sont compatibles avec le polymère gélifiant utilisé pour structurer la phase huileuse.

Les huiles non volatiles peuvent, notamment, être choisies parmi les huiles hydrocarbonées, le cas échéant fluorées et/ou les huiles siliconées non volatiles.

Comme huile hydrocarbonée non volatile, on peut notamment citer :

- les huiles hydrocarbonées d'origine animale, telle que le squalane ;
- les huiles hydrocarbonées d'origine végétale telles que les esters de phytostéaryle, comme l'oléate de phytostéaryle, l'isostéarate de phystostéaryle et le glutamate de lauroyl/octyldodécyle/phytostéaryle (AJINOMOTO, ELDEW PS203) ; les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées, ces huiles sont notamment des triglycérides héptanoïques ou octanoïques ; les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STÉARINERIES DUBOIS ou ceux vendus sous les dénominations MIGLYOL 810®, 812® et 818® par la société DYNAMIT NOBEL et leurs mélanges,
- les hydrocarbures linéaires ou ramifiés, d'origine minérale ou synthétique tels que la vaseline, les polydécènes, le polyisobutène hydrogéné tel que le parléam, et leurs mélanges ;
- les éthers de synthèse ayant de 10 à 40 atomes de carbone;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$ ;
- et leurs mélanges.

Les esters peuvent être notamment choisis parmi les esters d'acide gras comme par exemple :

- l'octanoate de cétostéaryle, les esters de l'alcool isopropylique, tels que le myristate d'isopropyle, le palmitate d'isopropyle, le lauroylsarcosinate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, les octanoates, décanoates ou ricinoléates d'alcools ou de polyalcools comme le dioctanoate de propylène glycol, l'octanoate de cétyle, l'octanoate de tridécyle, le 4-diheptanoate d'éthyle 2-hexyle, le diheptanoate de polyéthylène glycol, le diétyl 2-d'hexanoate de propylèneglycol et leurs mélanges, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle, le benzoate d'alkyle, les benzoates d'alcools en $C_{12}$ à $C_{15}$, et leurs mélanges ;

- les esters de polyols et les esters du pentaérythritol, comme le tétrahydroxystéarate/tétraisostéarate de dipentaérythritol ;
- les esters de dimères diols et dimères diacides tels que les Lusplan DD-DA5® et DD-DA7®, et leurs mélanges, commercialisés par la société NIPPON FINE CHEMICAL et décrits dans la demande FR 0 302 809 déposée le 6 mars 2003, dont le contenu est incorporé dans la présente demande par référence ;
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol ;
- les acides gras supérieurs liquides tels que l'acide oléique, l'acide linoléique, l'acide linolénique, et leurs mélanges ;
- les carbonates de di-alkyle, les 2 chaînes alkyles pouvant être identiques ou différentes, tel que le dicaprylyl carbonate commercialisé sous la dénomination CETIOL CC®, par COGNIS ; et
- leurs mélanges.

Les huiles de silicone non volatiles utilisables dans la composition selon l'invention peuvent être les polydiméthylsiloxanes (PDMS) non volatiles, comme la siméthicone, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, et les 2-phényléthyltriméthylsiloxysilicates, les diméthicones ou phényltriméthicone de viscosité inférieure ou égale à 100 cst, et leurs mélanges.

Les huiles hydrocarbonées volatiles optionnellement présentes peuvent être choisies, notamment, parmi les huiles hydrocarbonées ayant de 8 à 16 atomes de carbone, et notamment les alcanes ramifiés en $C_8$-$C_{16}$ (appelées aussi isoparaffmes), comme l'isododécane (encore appelé 2,2,4,4,6-pentaméthylheptane), l'isodécane, l'isohexadécane, et par exemple les huiles vendues sous les noms commerciaux d'ISOPARS® ou de PERMETHYLS®.

Comme huiles volatiles optionnellement présentes, on peut aussi utiliser les silicones volatiles, comme par exemple les huiles de silicones linéaires ou cycliques volatiles, notamment celles ayant une viscosité ≤ 8 centistokes ($8 \times 10^{-6}$ $m^2/s$), et ayant notamment de 2 à 10 atomes de silicium, et en particulier de 2 à 7 atomes de silicium. Ces huiles de silicone comportent éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt comme l'octaméthylcyclotétrasiloxane, le décaméthylcyclopentasiloxane, le dodécaméthylcyclohexasiloxane, l'heptaméthylhexyl-trisiloxane, l'heptaméthyloctyltrisiloxane, l'hexaméthyldisiloxane, l'octaméthyltrisiloxane, le décaméthyltétrasiloxane, le dodécaméthylpentasiloxane, et leurs mélanges.

On peut également utiliser des huiles volatiles fluorées telles que le nonafluorométhoxybutane ou le perfluorométhylcyclopentane, et leurs mélanges.

De manière avantageuse, la phase huileuse présente dans les particules conformes à la présente invention, peut comprendre au moins une huile notamment choisie parmi le squalane, l'isononanoate d'isononyle, le lauroylsarcosinate d'isopropyle, l'octyldodécanol, et leurs mélanges.

La teneur en phase huileuse des particules conformes à l'invention peut varier de 10 à 99 % en poids, en particulier d'au moins 20 à 99 % en poids, plus particulièrement de 30 à 99 % en poids, par rapport au poids total de la particule calibrée selon l'invention.

Selon un mode de réalisation, la phase huileuse, structurée par au moins un polymère gélifiant formant des particules huileuses calibrées conformes à l'invention, possède un point de fusion supérieur ou égal à 40 °C, de préférence inférieur ou égal à 95 °C.

Selon un mode de réalisation préféré, la même phase huileuse possède un point de fusion pouvant varier de 50 à 90 °C.

La phase huileuse structurée dont dérivent les particules huileuses calibrées conformes à l'invention possède avantageusement une viscosité supérieure à 750 Pa.s au cisaillement de 1 $s^{-1}$ à 25 °C, de préférence inférieure ou égale à $1.10^6$ Pa.s.

On peut faire la mesure au rhéomètre RFS3 Rhéométrics.

Les mesures sont alors faites à 25 °C, la température étant régulée par effet Peltier.

La géométrie est un cône / plan, cône de 25 mm de diamètre et angle 2°.

On impose un gradient de vitesse de 1 $s^{-1}$, pendant un certain temps d'équilibre, 5 minutes par exemple. La viscosité est donnée en Pa.s, à température et temps donnés.

La viscosité est en réalité mesurée à un stade où les particules ne sont pas encore formées, à savoir au niveau du pré-mélange huile, gélifiant et actif, comme cela ressort à la lecture du procédé détaillé ci-après.

Le choix de l'huile ou des huiles rentrant dans la formulation de la phase huileuse, ainsi que celui du ou des polymère(s) gélifiant(s) peut être ajusté par l'homme du métier, de manière à ce que la phase huileuse structurée des particules conformes à l'invention, réponde aux critères de point de fusion et de viscosité précédemment décrits.

## POLYMÈRE GÉLIFIANT

Les particules huileuses calibrées et sphériques sont avantageusement obtenues à partir d'au moins une phase huileuse structurée par au moins un polymère gélifiant choisi notamment parmi un polymère semi-cristallin, un monoalkyl- ou polyalkyl-ester de polysaccharide, un polyamide, un polyamide siliconé, un copolymère di-bloc, tri-bloc, multi-bloc et/ou radial-bloc, et leurs mélanges.

Par "polymère", on entend au sens de la présente invention désigner des composés comportant au moins deux motifs de répétition, de préférence au moins trois motifs de répétition et notamment au moins 10 motifs de répétition.

La teneur en polymère(s) gélifiant(s), dans les particules conformes à l'invention, peut varier de 1 à 80 % en poids, voire de 1 à 60 % en poids par rapport au poids total de la particule.

Avantageusement, le rapport pondéral entre le polymère gélifiant et la phase huileuse de la particule selon l'invention peut varier de 0,01 à 4, voire de 0,05 à 2, et en particulier de 0,1 à 1.

*Polymère semi-cristallin*

Par "polymère semi-cristallin", on entend au sens de l'invention, des polymères comportant une partie cristallisable, chaîne pendante ou séquence dans le squelette, et une partie amorphe dans le squelette et présentant une température de changement de phase réversible du premier ordre, en particulier de fusion (transition solide-liquide). Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

Les polymères semi-cristallins utilisables pour la mise en oeuvre de la présente invention sont solides à température ambiante et présentent de préférence une température de fusion (ou gélification) inférieure à 80 °C.

Ils comportent :

a) un squelette polymérique, et
b) au moins une chaîne organique latérale cristallisable et/ou une séquence organique cristallisable faisant partie du squelette dudit polymère, ledit polymère ayant une masse moléculaire moyenne en nombre supérieure ou égale à 2 000.

De façon avantageuse, le ou les polymères semi-cristallins de la composition de l'invention ont une masse moléculaire moyenne en nombre Mn supérieure ou égale à 2000, allant par exemple de 2 000 à 800 000, notamment de 3 000 à 500 000.

Selon un mode de réalisation, les polymères semi-cristallin utilisables dans le cadre de l'invention présentent une température de fusion (ou point de fusion) pF inférieure à 70 °C, notamment inférieure à 50 °C. Le polymère semi-cristallin a avantageusement une température de fusion pF comprise dans l'intervalle allant de 40 °C à moins de 80 °C. En réalité, le polymère semi-cristallin peut être un mélange de polymères semi-cristallin. Dans ce cas, c'est le mélange qui présente une température de fusion pF comprise dans ledit intervalle. Autrement dit, le mélange peut comprendre un polymère semi-cristallin présentant un point de fusion en dehors de cet intervalle, pour autant que le mélange, quant à lui, présente une température de point de fusion dans ledit intervalle. La température de fusion peut être mesurée notamment par toute méthode connue et en particulier avec un calorimètre à balayage différentiel (D.S.C).

Selon une variante de réalisation, les séquences ou chaînes cristallisables des polymères semi-cristallins représentent au moins 30 %, voire au moins 40 % du poids total de chaque polymère. Les polymères semi-cristallins de l'invention à séquences cristallisables peuvent être des polymères séquencés ou multiséquencés. Ils peuvent être obtenus par polymérisation de monomères à doubles liaisons réactives (liaisons éthyléniques) ou par polycondensation. Lorsque les polymères de l'invention sont des polymères à chaînes latérales cristallisables, ceux-ci sont avantageusement sous forme aléatoire ou statistique.

Les polymères semi-cristallins utilisables dans l'invention sont, par exemple :

1. les copolymères séquencés de polyoléfmes à cristallisation contrôlée, dont les monomères sont décrits dans le document EP-A-951897 ;
2. les polycondensats et notamment les polycondensats polyesters, aliphatiques ou aromatiques, et les copolyesters aliphatiques/aromatiques ;
3. les homo- ou co-polymères portant au moins une chaîne latérale cristallisable et les homo- ou co-polymères portant dans le squelette au moins une séquence cristallisable, comme ceux décrits dans le brevet US-A-5,156,911 ;
4. les homo- ou co-polymères portant au moins une chaîne latérale cristallisable à groupement(s) fluoré(s), tels que décrits dans la demande WO-A-01/19333 ;
5. et leurs mélanges.

Dans les deux derniers cas (3 et 4), la ou les chaînes latérales ou séquences cristallisables sont hydrophobes.

Les polymères cristallins à chaînes latérales cristallisables, ou portant dans le squelette au moins une séquence cristallisable convenant à la mise en oeuvre de l'invention, sont par exemple, décrits ci-dessous.

*A) Polymères semi-cristallins à chaînes latérales cristallisables*

On peut citer notamment les polymères définis dans les documents US 5 156 911 et WO-A-01/19333. Ce sont des homopolymères ou copolymères comportant de 50 à 100 % en poids de motifs résultant de la polymérisation d'un ou de plusieurs monomères porteurs de chaîne(s) latérale(s) hydrophobe(s) cristallisable(s).

Ces homo- ou co-polymères sont de toute nature du moment qu'ils présentent les conditions indiquées ci-après avec, en particulier, la caractéristique d'être solubles ou dispersables dans la phase huileuse par chauffage au-dessus de leur température de fusion pF (ou gélification). Ils peuvent résulter :

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique ;
- de la polycondensation d'un ou plusieurs monomères porteurs de groupes coréactifs (acide carboxylique ou sulfonique, alcool, amine ou isocyanate), comme par exemple les polyesters, les polyuréthanes, les polyéthers, les polyurées, les polyamides.

D'une façon générale les motifs (chaînes ou séquences) cristallisables des polymères semi-cristallins utilisables dans le cadre de l'invention, proviennent de monomère(s) à séquence(s) ou chaîne(s) cristallisable(s), utilisé(s) pour la fabrication des polymères semi-cristallins. Ces polymères sont choisis, notamment, parmi les homopolymères et copolymères résultant de la polymérisation d'au moins un monomère à chaîne(s) cristallisable(s) qui peut être représenté par la formule X :

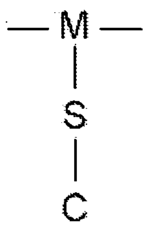

dans laquelle M représente un atome du squelette polymérique, S représente un espaceur et C représente un groupe cristallisable.

Les chaînes "-S-C" cristallisables peuvent être aliphatiques ou aromatiques, éventuellement fluorées ou perfluorées. "S" représente notamment un groupe $(CH_2)n$ ou $(CH_2CH_2O)n$ ou $(CH_2O)$, linéaire ou ramifié ou cyclique, n étant un nombre entier allant de 0 à 22. De préférence "S" est un groupe linéaire. De préférence, "S" et "C" sont différents.

Lorsque les chaînes cristallisables sont des chaînes aliphatiques (alkyle), elles comportent au moins 11 atomes de carbone et au plus 40 atomes de carbone et mieux au plus 24 atomes de carbone. Il s'agit notamment de chaînes alkyle possédant au moins 12 atomes de carbone, et par exemple, il peut s'agir de chaînes alkyle comportant de 14 à 24 atomes de carbone ($C_{14}$-$C_{24}$). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.

Comme exemples de polymères ou copolymères semi-cristallins à chaîne(s) cristallisable(s), on peut citer ceux résultant de la polymérisation d'au moins un monomère à chaîne cristallisable choisi parmi les (méth)acrylates d'alkyle saturés en $C_{14}$-$C_{24}$ ($C_{14}$-$C_{24}$ signifie que le groupe alkyle comporte de 14 à 24 atomes de carbone) ; les (méth)acrylates de perfluoroalkyle en $C_{11}$-$C_{15}$ (groupe alkyle avec 11 à 15 atomes de carbone) ; les N-alkyl(méth)acrylamides en $C_{14}$ à $C_{24}$ avec ou sans atome de fluor ; les esters vinyliques à chaînes alkyle ou perfluoroalkyle en $C_{14}$ à $C_{24}$, avec une chaîne perfluoroalkyle comportant au moins 6 atomes de fluor ; les éthers vinyliques à chaînes alkyle ou perfluoroalkyle en $C_{14}$ à $C_{24}$, avec une chaîne perfluoroalkyle comportant au moins 6 atomes de fluor ; les alpha-oléfines en $C_{14}$ à $C_{24}$, comme par exemple l'octadécène ; les para-alkyl styrènes en $C_{14}$ à $C_{24}$, leurs mélanges.

Par "alkyle", on entend au sens de l'invention un groupement saturé notamment, comportant de 8 à 24 atomes de carbone ($C_8$ à $C_{24}$), sauf mention exprès.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un

diisocyanate.

Lorsque les polymères utilisés dans la composition de l'invention sont des copolymères, ils contiennent, en plus, de 0 à 50 % de groupes Y ou Z résultant de la copolymérisation :

α) avec Y qui est un monomère polaire ou non polaire ou un mélange des deux :

- lorsque Y est un monomère polaire, c'est soit un monomère porteur de groupes polyoxyalkylénés (notamment oxyéthylénés et/ou oxypropylénés), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrrolidone (NVP), le N-vinyl-caprolactame, soit un monomère porteur d'au moins un groupe acide carboxylique comme les acides (méth)acryliques, crotonique, itaconique, maléique, fumarique ou porteur d'un groupe anhydride d'acide carboxyliquecomme l'anhydre maléique, et leurs mélanges.
- lorsque Y est un monomère non polaire, il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle comportant de 1 à 10 atomes de carbone (Ci à $C_{10}$), comme l'α-méthylstyrène, un macromonomère du type polyorganosiloxane à insaturation vinylique.

β) avec Z qui est un monomère polaire ou un mélange de monomères polaires, Z ayant la même définition que le "Y polaire" défmi ci-dessus.

De préférence, les polymères semi-cristallins à chaîne latérale cristallisable sont choisis parmi les homopolymères d'alkyl(méth)acrylate ou d'alkyl(méth)acrylamide avec un groupe alkyle tel que défini ci-dessus, et notamment en $C_{14}$-$C_{24}$ ; les copolymères de ces monomères avec un monomère hydrophile de préférence de nature différente de l'acide (méth)acrylique ; et leurs mélanges. Il peut s'agir, par exemple, comme copolymères, des copolymères d'alkyl (méth)acrylate ou d'alkyl (méth)acrylamide avec un groupe alkyle en $C_{14}$ à $C_{24}$, avec la N-vinylpyrrolidone, l'hydroxyéthyl (méth)acrylate ; ou leurs mélanges.

*B) Les polymères portant dans le squelette au moins une séquence cristallisable*

Il s'agit encore de polymères solubles ou dispersables dans l'huile ou phase huileuse par chauffage au-dessus de leur point de fusion pF. Ces polymères sont notamment des copolymères séquencés constitués d'au moins 2 séquences de nature chimique différente dont l'une est cristallisable.

A titre de polymères portant dans le squelette au moins une séquence cristallisable convenant à la mise en oeuvre de l'invention, on peut mentionner:

1. Les polymères définis dans le document US-A-5,156,911 ;
2. Les copolymères séquencés d'oléfme ou de cyclooléfine à chaîne cristallisable comme ceux issus de la polymérisation séquencée de :

- cyclobutène, cyclohexène, cyclooctène, norbornène (c'est-à-dire bicyclo(2,2,1)heptène 2), 5-méthylnorbornène, 5-éthylnorbornène, 5,6-diméthyl-norbomène, 5,5,6-triméthyl norbornène, 5-éthylidène-norbornène, 5-phénylnorbornène, 5-benzylnorbornène, 5-vinylnorbornène, 1,4,5,8-diméthano-1,2,3,4,4a,5,8a-octahydronaphtalène, dicyclopentadiène, et leurs mélanges ;
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges. Ces copolymères séquencés peuvent être en particulier les copolymères (éthylène/norbornène) blocs et les terpolymères blocs (éthylène/propylène/ éthylidène-norbomène).

On peut aussi utiliser ceux résultants de la copolymérisation séquencée d'au moins 2 α-oléfines en $C_2$-$C_{16}$ et mieux en $C_2$-$C_{12}$, tels que ceux cités précédemment et en particulier les bipolymères séquencés d'éthylène et d'1-octène.
3. Les copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :

- Séquence cristallisable par nature : a) polyester comme les poly(alkylène téréphtalate), b) polyoléfine comme

les polyéthylènes ou polypropylènes.

- Séquence amorphe et lipophile comme les polyoléfines ou copoly(oléfme)s amorphes telles que le poly(isobutylène), le polybutadiène hydrogéné, le poly(isoprène) hydrogéné.

Comme exemple de tels copolymères à séquence cristallisable et à séquence amorphe, on peut citer :

α) les copolymères séquencés poly(Σ-caprolactone)-b-poly(butadiène), utilisés de préférence hydrogénés, tels que ceux décrits dans l'article "Melting behavior of poly(Σ -caprolactone)-block-polybutadiène copolymers" de S. Nojima, Macromolécules, 32, 3727-3734 (1999).

β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).

γ) les copolymères séquencés poly(éthylène)-b-copoly(éthylène/propylène) cités dans les articles "Morphology of semi-crystalline block copolymers of ethylene- (ethylene-alt-propylene)" de P. Rangarajan et al., Macromolecules, 26, 4640-4645 (1993), et "Polymer agregates with crystalline cores : the system poly(ethylene)- poly(ethylene-propylene)" P. Richter et al., Macromolécules, 30, 1053-1068 25 (1997).

δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins utilisables dans le cadre de l'invention peuvent être non réticulés ou réticulés en partie, du moment que le taux de réticulation ne gêne pas leur dissolution ou dispersion dans la phase huileuse liquide par chauffage au dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins convenant à l'invention sont non réticulés.

A titre d'exemple particulier de polymère semi-cristallin utilisable dans la composition selon l'invention, on peut citer les produits INTELIMER® de la société LANDEC décrits dans la brochure "INTELIMER® POLYMERS". Ces polymères sont sous forme solide à température ambiante (25 °C). Ils sont porteurs de chaînes latérales cristallisables et présentent le monomère tel que défini dans la formule X précédente. On peut citer notamment le "LANDEC IP22®", ayant une température de fusion pF de 56 °C, qui est un produit visqueux à température ambiante, imperméable, non-collant.

On peut aussi utiliser le polymère "STRUCTURE O" commercialisé par la société NATIONAL STARCH, tel que celui décrit dans le document US-A-5,736,125, de pF 44 °C, ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP ou par copolymérisation de l'acrylate de béhényle et de l'acide acrylique ou de NVP, tels que décrits dans le document US-A-5,519,063 ou EP-A- 0550745.

Selon une variante particulière de réalisation, les polymères semi-cristallins convenant à la mise en oeuvre de la présente invention sont notamment les acrylates alkylés, parmi lesquels on peut mentionner les copolymères de LANDEC :

- DORESCO IPA 13-1® : poly acrylate de stéaryle, pf de 49 °C et PM de 145000;
- DORESCO IPA 13-3® : poly acrylate/ acide méthacrylique, pf de 65 °C et PM de 114000;
- DORESCO IPA 13-4® : poly acrylate/ vinyl pirrolidone, pf de 44 °C et PM de 387000;
- DORESCO IPA13-5® : poly acrylate / methacrylate d'hydroxyethyle, pf de 47 °C et PM de 397600;
- DORESCO IPA 13-6® : poly acrylate de béhényle, pf de 66 °C.

*Les polyamides*

Les polyamides avantageusement utilisables dans la préparation des particules selon la présente invention sont, notamment, ceux décrits dans le document US-A-5 783 657 de la Société UNION CAMP.

Les polyamides convenant à la mise en oeuvre de l'invention satisfont notamment à la formule suivante :

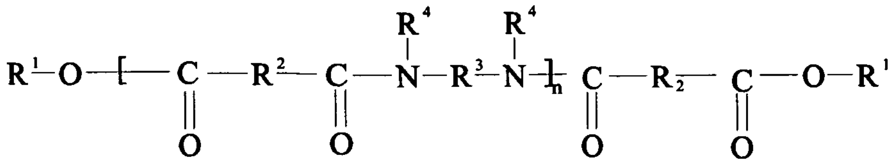

dans laquelle :

- n désigne un nombre entier de motifs amide tel que le nombre de groupes ester représente de 10 % à 50 % du nombre total des groupes ester et amide ;
- $R^1$ est à chaque occurrence, indépendamment, un groupe alkyle ou alcényle ayant au moins 4 atomes de carbone et notamment de 4 à 24 atomes de carbone ;
- $R^2$ représente à chaque occurrence, indépendamment, un groupe hydrocarboné en $C_4$ à $C_{55}$ à condition que 50 % au moins des groupes $R^2$ représentent un groupe hydrocarboné en $C_{30}$ à $C_{55}$;
- $R^3$ représente à chaque occurrence, indépendamment, un groupe organique pourvu d'au moins 2 atomes de carbone, d'atomes d'hydrogène et optionnellement d'un ou plusieurs atomes d'oxygène ou d'azote ; et
- $R^4$ représente à chaque occurrence, indépendamment, un atome d'hydrogène, un groupe alkyle en $C_1$ à $C_{10}$ ou une liaison directe à $R^3$ ou à un autre $R^4$ de sorte que l'atome d'azote auquel sont liés à la fois $R^3$ et $R^4$ fasse partie d'une structure hétérocyclique définie par $R^4$-N-$R^3$, avec au moins 50 % des $R^4$ représentant un atome d'hydrogène.

Selon une variante de réalisation, les groupes esters de ces polyamides représentent de 15 à 40 % du nombre total des groupes ester et amide et au mieux de 20 à 35 %. De plus, n représente avantageusement un nombre entier allant de 1 à 10, et mieux de 1 à 5.

Notamment, $R^1$ est un groupe alkyle en $C_{12}$ à $C_{22}$, voire en $C_{16}$ à $C_{22}$. $R^2$ peut être notamment un groupe hydrocarboné (alkylène) en $C_{10}$ à $C_{42}$. Notamment, 50 % au moins, et mieux 75 % au moins des $R^2$ peuvent être des groupes ayant de 30 à 42 atomes de carbone. Les autres $R^2$ sont des groupes hydrogénés en $C_4$ à $C_{19}$, et en particulier en $C_4$ à $C_{12}$. $R^3$ peut représenter un groupe hydrocarboné en $C_2$ à $C_{36}$ ou un groupe polyoxyalkyléné et $R^4$ représente un atome d'hydrogène. Notamment, $R^3$ peut représenter un groupe hydrocarboné en $C_2$ à $C_{12}$. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

A titre d'exemple de polyamide structurant utilisable dans l'invention, on peut encore citer les résines polyamides résultant de la condensation d'un acide di-carboxylique aliphatique et d'un diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque VERSAMID par les sociétés GENERAL MILLS, Inc. et HENKEL CORP., sous la marque ONAMID notamment ONAMID S ou C. Ces résines ont une masse moléculaire moyenne en poids allant de 6000 à 9000. Les documents US-A-3 645 705 et US-A-3 148 125 décrivent ces résines. Selon une variante de réalisation, on utilise les VERSAMID 930 ou 744.

On peut aussi utiliser les polyamides vendus ou fabriqués par la société ARIZONA sous les références UNI-REZ (2658, 2931, 2970, 2621,2613, 2624, 2665, 1554, 2623, 2662) et le produit vendu sous la référence MACROMELT 6212® par la société HENKEL. Le brevet US-A-5500209 décrit ce type de polymères.

A titre d'exemple de polyamides structurant utilisables dans la composition selon l'invention, on peut encore citer les produits commerciaux vendus ou fabriqués par la société ARIZONA CHEMICAL sous les noms UNICLEAR 80® et UNICLEAR 100®. Ils sont vendus respectivement sous forme de gel à 80 % (en matière active) et à 100 % (en matière active) dans une huile minérale. Ils ont un point de ramollissement de 88 à 105 °C. Ces produits commerciaux sont un mélange de copolymère d'un diacide en $C_{36}$ condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

Les polyamides structurant ont avantageusement une température de ramollissement supérieure à 60 °C et pouvant aller jusqu'à 190 °C. De préférence, ils présentent une température de ramollissement inférieure à 150 °C allant de 70 à 130 °C et mieux de 80 °C à 105 °C.

La structuration de la phase huileuse peut être obtenue à l'aide d'un ou plusieurs polyamides défmis ci-dessus. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Les polyamides utilisés dans la présente demande présentent du fait de leur chaîne grasse, une bonne solubilité dans la phase huileuse et donc conduisent à des compositions macroscopiquement homogènes même avec un taux élevé de polymère.

A titre d'exemple de polyamide convenant à la mise en oeuvre de la présente invention, on peut mentionner le copolymère d'éthylène diamide/stéaryle dimèrdilinoléate, commercialisé sous la référence UNICLEAR 100® VG, par la société ARIZONA CHEMICAL.

*Polyamides siliconés*

Les polymères (homopolymères ou copolymères) de type polyamide, convenant à la mise en oeuvre de l'invention, possèdent une masse moléculaire moyenne comprise dans l'intervalle allant de 500 à 500 000, et possèdent au moins un groupe comprenant :

- au moins un groupe polyorganosiloxane, comportant de 1 à 1000 unités organosiloxane, dans la chaîne du groupe ou sous forme de greffon, et
- au moins deux groupes capables d'établir des interactions hydrogènes choisis parmi les groupes ester, amide, sulfonamide, carbamate, thiocarabamate, urée, thiourée, oxamido, guanidino, biguanidino, et leurs combinaisons, à condition qu'au moins un de ces groupes soit différent d'un groupe ester, le polymère étant solide à température ambiante et soluble dans la phase huileuse à une température variant de 25 à 150 °C. En particulier, le polymère est soluble dans la phase huileuse à une température variant de 41 à 120 °C.

Les polymères convenant à la mise en oeuvre de l'invention, et utilisés comme agent gélifiant de l'huile, peuvent appartenir aux deux familles suivantes :

- des polyorganosiloxanes comprenant au moins deux groupes capables d'établir des interactions hydrogènes, ces deux groupes étant disposés dans la chaîne du polymère, et/ou,
- des polyorganosiloxanes comprenant au moins deux groupes capables d'établir des interactions hydrogènes, ces deux groupes étant disposés sur des greffons ou ramifications.

Les polymères comportant deux groupes capables d'établir des interactions hydrogène dans la chaîne du polymère peuvent être des polymères comprenant au moins un motif répondant à la première formule suivante :

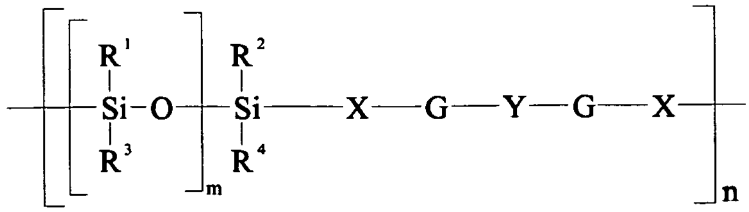

dans laquelle :

1. $R^1$, $R^2$, $R^3$ et $R^4$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote ;

2. les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote ;
3. Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4. Y représente un groupe répondant à la formule :

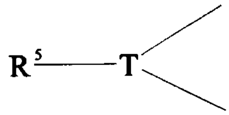

dans laquelle :

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
  $R^5$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5. les G, identiques ou différents, représentent les groupes divalents choisis parmi :

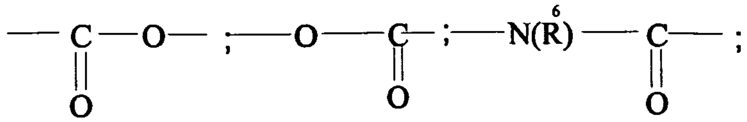

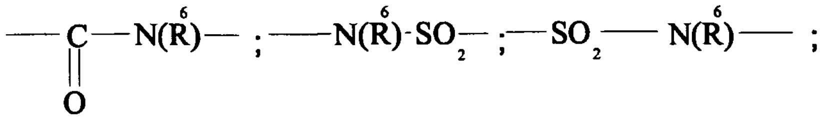

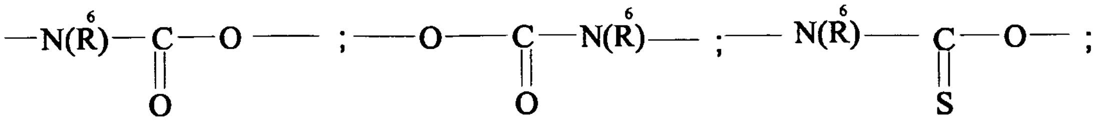

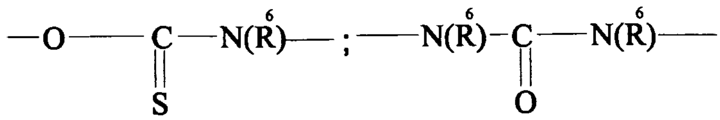

et

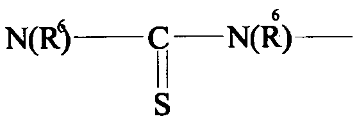

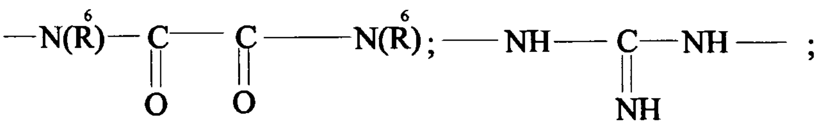

et

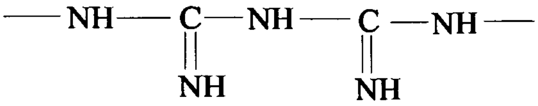

où $R^6$ représente un atome d'hydrogène ou un groupe alkyle, linéaire ou ramifié, en $C_1$ à $C_{20}$.

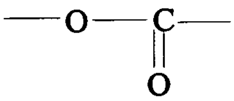 et 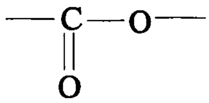 ;

5. n est un nombre entier allant de 2 à 500, en particulier de 2 à 200, et m est un nombre entier allant de 1 à 1000, en particulier de 1 à 700 et mieux encore de 6 à 200.

Selon une variante de réalisation, 80 % des $R^1$, $R^2$, $R^3$ et $R^4$, du polymère peuvent être choisis notamment parmi les groupes méthyle, éthyle, phényle et 3,3,3-trifluoropropyle.

Selon une autre variante de réalisation, Y peut représenter divers groupes divalents, comportant éventuellement de plus une ou deux valences libres pour établir des liaisons avec d'autres motifs du polymère ou copolymère. Notamment, Y peut représenter un groupe choisi parmi :

a) les groupes alkylène linéaires en $C_1$ à $C_{20}$, notamment en $C_1$ à $C_{10}$;
b) les groupes alkylène ramifiés pouvant comporter des cycles et des insaturations non conjuguées, en $C_{30}$ à $C_{56}$ ;
c) les groupes cycloalkylène en $C_5$-$C_6$ ;
d) les groupes phénylène éventuellement substitués par un ou plusieurs groupes alkyle en $C_1$ à $C_{40}$ ;
e) les groupes alkylène en $C_1$ à $C_{20}$, comportant de 1 à 5 groupes amides;
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$ ;
g) les chaînes polyorganosiloxane de formule:

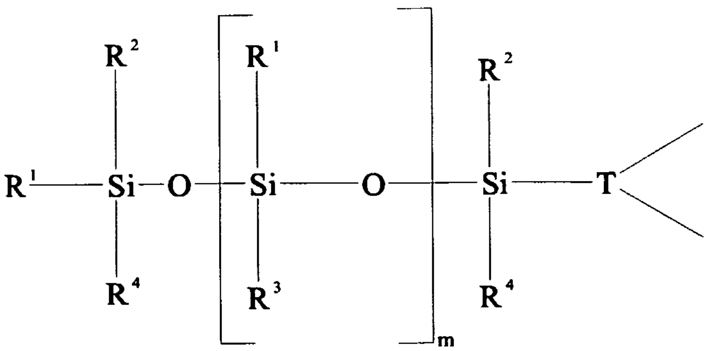

dans laquelle $R^1$, $R^2$, $R^3$ et $R^4$, T et m sont tels que définis ci-dessus; et
h) les chaînes polyorganosiloxanes de formule :

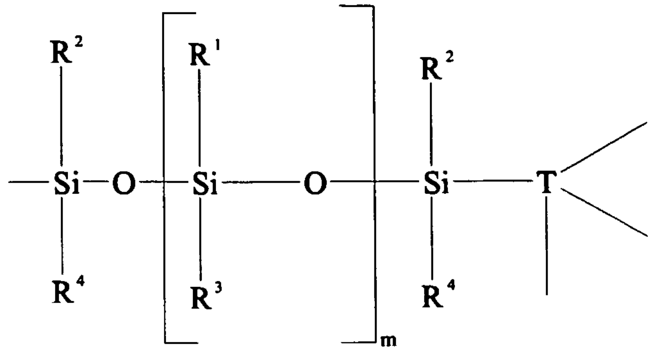

Les polyorganosiloxanes de la seconde famille peuvent être des polymères comprenant au moins un motif répondant à la seconde formule suivante :

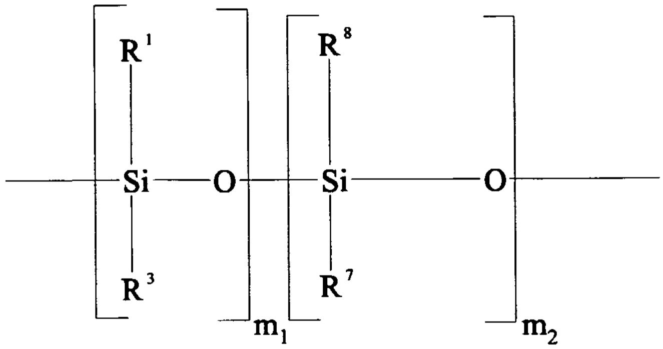

(II)

dans laquelle :

- $R^1$ et $R^3$, identiques ou différents, sont tels que définis ci-dessus pour la formule précédente ;
- $R^7$ représente un groupe tel que défmi ci-dessus pour $R^1$ et $R^3$, ou représente le groupe de formule $-X-G-R^9$ dans laquelle X et G sont tels que définis ci-dessus pour la formule précédente et $R^9$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$;
- $R^8$ représente le groupe de formule $-X-G-R^9$ dans laquelle X, G et $R^9$ sont tels que définis ci-dessus ;
- $m_1$ est un nombre entier allant de 1 à 998 ; et
- $m_2$ est un nombre entier allant de 2 à 500.

Selon l'invention, le polyamide siliconé utilisé comme agent gélifiant, peut être un homopolymère, c'est-à-dire un polymère comportant plusieurs motifs identiques, en particulier des motifs selon les formules définies précédemment

Selon l'invention, on peut aussi utiliser un polyamide siliconé constitué par un copolymère comportant plusieurs motifs selon la première formule précédente différents, c'est-à-dire un polymère dans lequel l'un au moins des $R^1$, $R^2$, $R^3$, $R^4$, X, G, Y, m et n est différent dans l'un des motifs. Le copolymère peut être aussi formé de plusieurs motifs selon la seconde formule précédente, dans lequel l'un au moins des $R^1$, $R^3$, $R^7$, $R^8$, $m_1$ et $m_2$ est différent dans l'un au moins des motifs.

On peut encore utiliser un copolymère comportant au moins un motif selon la première formule et au moins un motif selon la seconde formule, les motifs selon la première formule et les motifs selon la seconde formule pouvant être identiques ou différents les uns des autres.

Selon une variante de l'invention, on peut encore utiliser un polyamide siliconé de type copolymère comprenant de plus au moins un motif hydrocarboné comportant deux groupes capables d'établir des interactions hydrogènes choisis parmi les groupes ester, amide, sulfonamide, carbamate, thiocarbamate, urée, thiourée et leurs combinaisons. Ces copolymères peuvent être des copolymères blocs, des copolymères séquencés ou des copolymères greffés.

Selon une variante de réalisation, les groupes capables d'établir des interactions hydrogènes sont des groupes

amides de formule -C(O)NH- et -HN-C(O)-. Dans ce cas, l'agent gélifiant peut être, par exemple, un polymère comprenant au moins un motif selon la troisième ou la quatrième formule suivante :

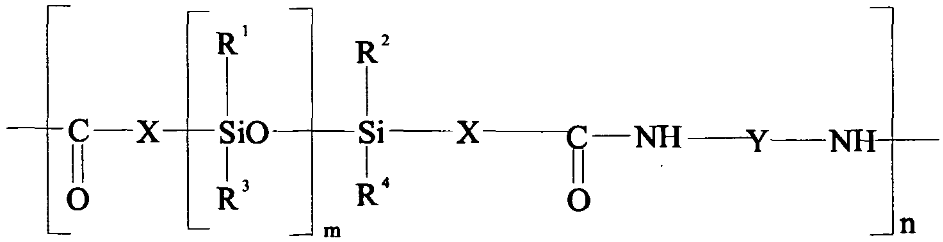

ou

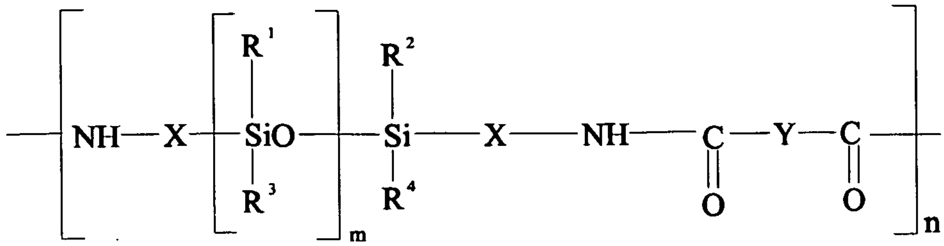

dans lesquelles $R^1$, $R^2$, $R^3$, $R^4$, X, Y, m et n sont tels que défmis précédemment.

Un tel motif peut être obtenu :

- soit par une réaction de condensation entre un silicone à extrémités α, ω-acides carboxyliques et une ou plusieurs diamines, selon le schéma réactionnel suivant :

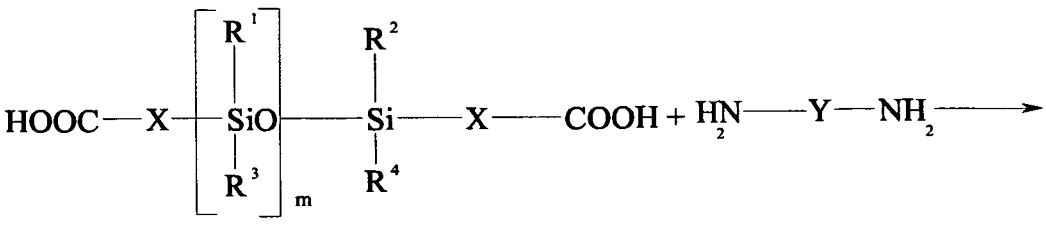

soit par réaction de deux molécules d'acide carboxylique alpha -insaturé avec une diamine selon le schéma réactionnel suivant : $CH_2=CH-X^1-COOH+H_2N-Y-NH_2$ ->$CH_2=CH-X^1$-CO-NH-Y-NH-CO-$X^1-CH=CH_2$ suivie de l'addition d'un siloxane sur les insaturations éthyléniques, selon le schéma suivant : $CH_2=CH-X^1$-CO-NH-Y-NH-CO-$X^1-CH=CH_2$ +

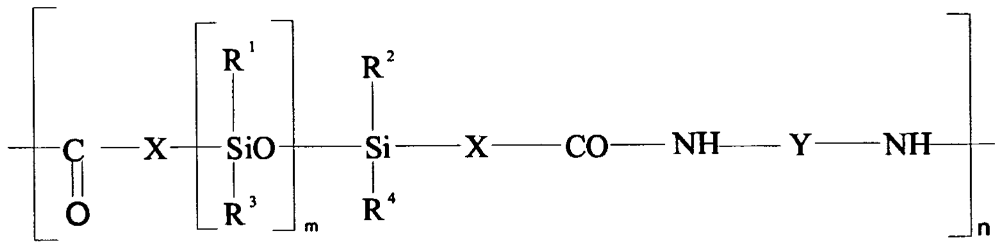

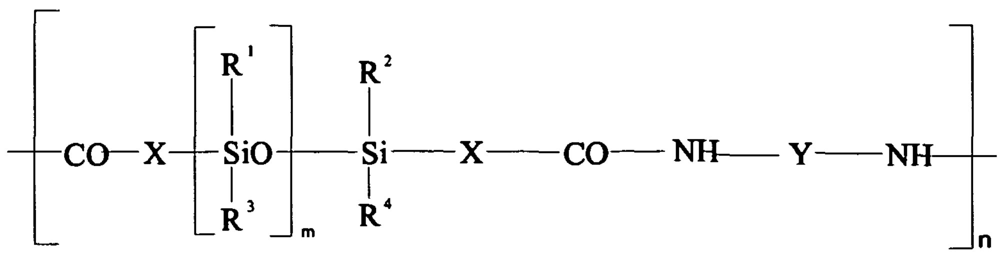

dans lesquels $X^1$-$(CH_2)_2$- correspond au X défini ci-dessus et Y, $R^1$, $R^2$, $R^3$, $R^4$ et m sont tels que définis précédemment ;

- soit par réaction d'un silicone à extrémités α, ω-NH2 et d'un diacide de formule HOOC-Y-COOH selon le schéma réactionnel suivant :

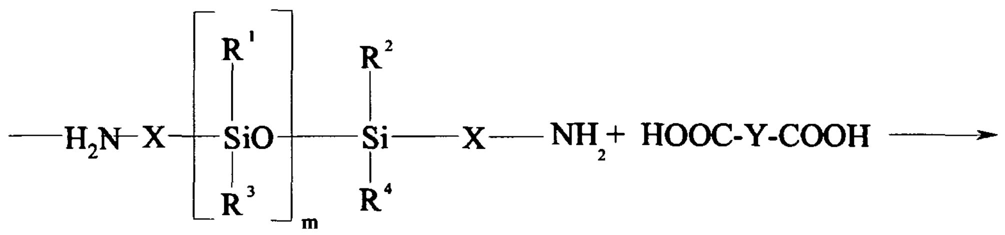

Dans les polyamides selon les troisième et quatrième formules précédemment exposées :

- m est notamment dans la gamme de 1 à 700, voire de 15 à 500 et mieux encore de 15 à 45, et
- n est en particulier dans la gamme de 1 à 500, notamment de 1 à 100 et mieux encore de 4 à 25,
- X est notamment une chaîne alkylène linéaire ou ramifiée ayant de 1 à 30 atomes de carbone, en particulier 3 à 10 atomes de carbone, et
- Y est notamment une chaîne alkylène linéaire ou ramifiée ou pouvant comporter des cycles et/ou des insaturations ayant de 1 à 40 atomes de carbone, en particulier de 1 à 20 atomes de carbone, et mieux encore de 2 à 6 atomes de carbone, en particulier de 6 atomes de carbone.

Dans les troisième et quatrième formules exposées précédemment, le groupe alkylène représentant X ou Y peut éventuellement contenir dans sa partie alkylène au moins l'un des éléments suivants :

1) 1 à 5 groupes amides, urée ou carbamate,
2) un groupe cycloalkyle en $C_5$ ou $C_6$, et
3) un groupe phénylène éventuellement substitué par 1 à 3 groupes alkyles identiques ou différents en $C_1$ à $C_3$.

Dans les troisième et quatrième formules exposées précédemment, les groupes alkylènes peuvent aussi être substitués par au moins un élément choisi dans le groupe constitué de :

- un groupe hydroxy,
- un groupe cycloalkyle en $C_3$ à $C_8$,
- un à trois groupes alkyles en $C_1$ à $C_{40}$,
- un groupe phényle éventuellement substitué par un à trois groupes alkyles en $C_1$ à $C_3$,
- un groupe hydroxyalkyle en $C_1$ à $C_3$, et
- un groupe aminoalkyle en $C_1$ à $C_6$. Dans les troisième et quatrième formules exposées précédemment, Y peut aussi représenter :

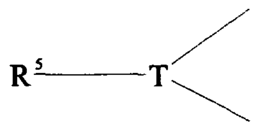

où $R^5$ représente une chaîne polyorganosiloxane, et T représente un groupe de formule :

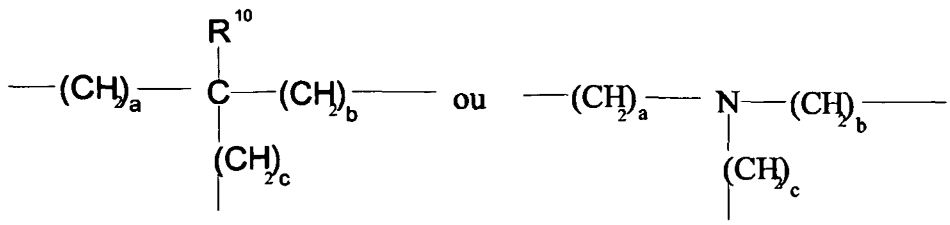

ou

dans lesquelles a, b et c sont, indépendamment, des nombres entiers allant de 1 à 10, et $R^{10}$ est un atome d'hydrogène ou un groupe tel que ceux définis pour $R^1$, $R^2$, $R^3$, $R^4$.

Dans les troisième et quatrième formules exposées précédemment, $R^1$, $R^2$, $R^3$, $R^4$ représentent notamment, indépendamment, un groupe alkyle en $C_1$ à $C_{40}$, linéaire ou ramifié, en particulier un groupe $CH_3$, $C_2H_5$, n-$C_3H_7$ ou isopropyle, une chaîne polyorganosiloxane ou un groupe phényle éventuellement substitué par un à trois groupes méthyle ou éthyle.

Comme on l'a vu précédemment, le polymère peut également comprendre des motifs selon la troisième ou quatrième formule exposées précédemment identiques ou différents.

Ainsi, le polymère peut être un polyamide siliconé contenant plusieurs motifs selon la troisième ou quatrième formule exposées précédemment de longueurs différentes, soit un polyamide répondant à la cinquième formule suivante :

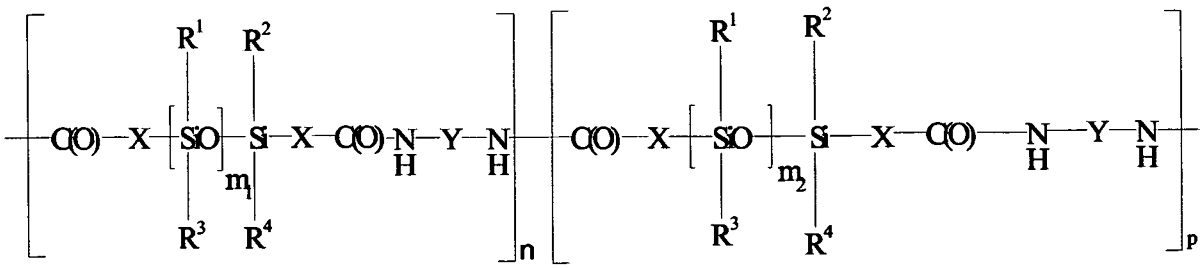

dans laquelle X, Y, n, $R^1$ à $R^4$ ont les significations données précédemment, $m_1$ et $m_2$ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le copolymère peut répondre à la sixième formule :

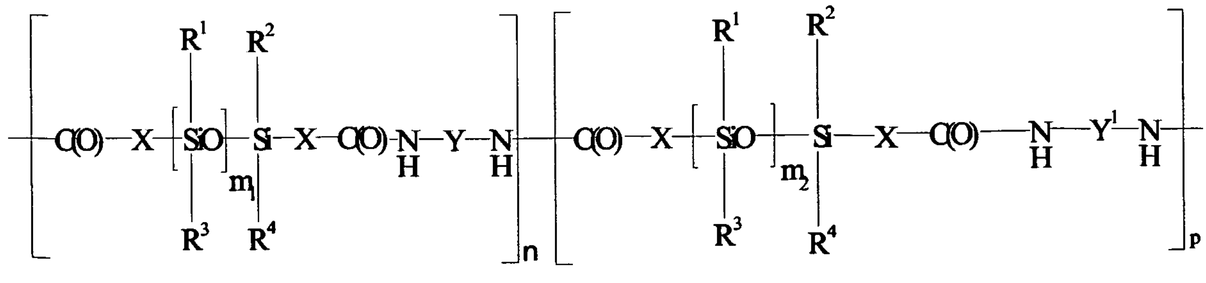

dans laquelle $R^1$ à $R^4$, X, Y, $m_1$, $m_2$, n et p ont les significations données ci-dessus et $Y^1$ est différent de Y, mais est

choisi parmi les groupes définis pour Y. Comme précédemment, les différents motifs peuvent être structurés pour former soit un copolymère bloc, soit un copolymère aléatoire, soit un copolymère alterné.

Selon un mode de réalisation de l'invention, le polyamide siliconé gélifiant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le copolymère peut comprendre au moins un motif selon la septième formule suivante :

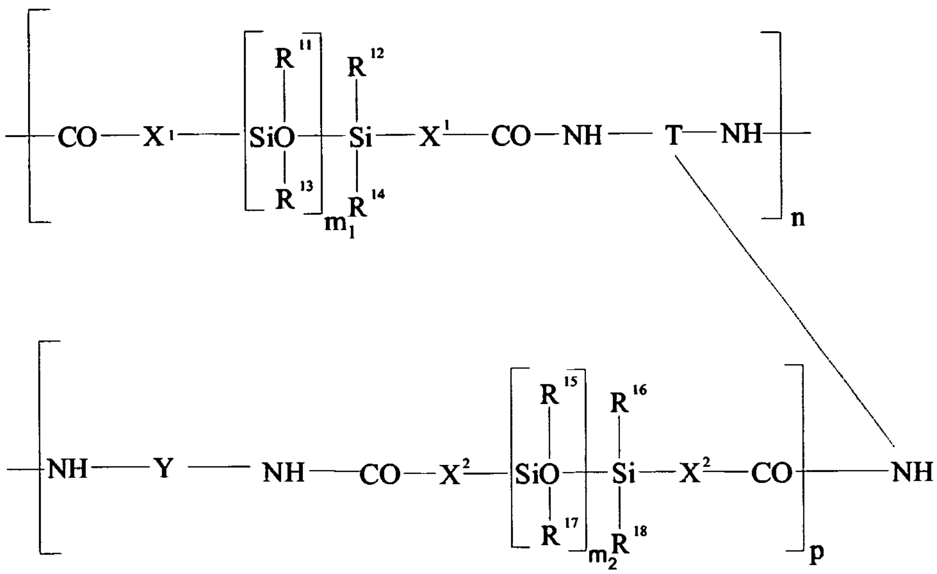

dans laquelle $X^1$ et $X^2$ qui sont identiques, ou différents, ont la signification donnée pour X dans la première formule précédente, n est tel que défini dans la première formule précédente, Y et T sont tels que définis dans la première formule précédente, $R^{11}$ à $R^{18}$ sont des groupes choisis dans le même groupe que les $R^1$ à $R^4$, $m_1$ et $m_2$ sont des nombres situés dans la gamme allant de 1 à 1 000, et p est un nombre entier allant de 2 à 500.

Dans la septième formule précédemment exposée, en particulier :

- p est dans la gamme de 1 à 25, mieux encore de 1 à 7,
- $R^{11}$ à $R^{18}$ sont des groupes méthyle,
- T répond à l'une des formules suivantes :

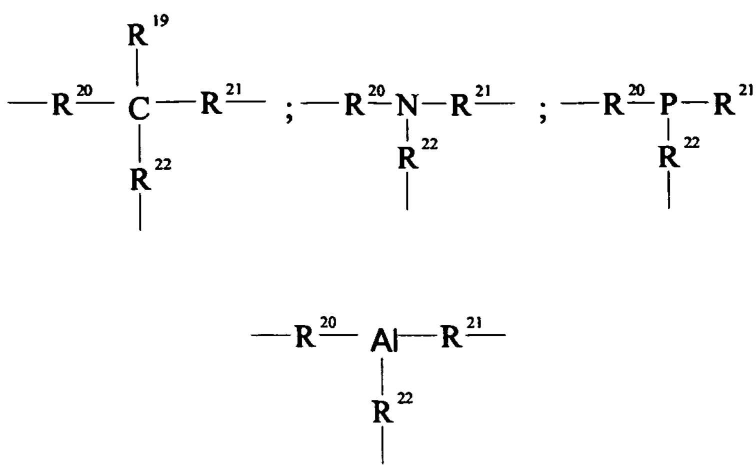

dans lesquelles $R^{19}$ est un atome d'hydrogène ou un groupe choisi parmi les groupes définis pour $R^1$ à $R^4$, et $R^{20}$, $R^{21}$ et $R^{22}$ sont, indépendamment, des groupes alkylène, linéaires ou ramifiés,

T répond en particulier de préférence à la formule :

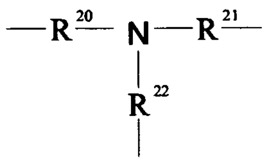

avec notamment $R^{20}$, $R^{21}$ et $R^{22}$ représentant $-CH_2-CH_2-$,

- $m_1$ et $m_2$ sont dans la gamme de 15 à 500, voire de 15 à 45,
- $-X^1$ et $X^2$ représentent $-(CH_2)_{10}-$, et
- Y représente $-CH_2-$.

Ces polyamides à motif silicone greffé selon la septième formule exposée précédemment peuvent être copolymérisés avec des polyamides-silicones selon la seconde formule pour former des copolymères blocs, des copolymères alternés ou des copolymères aléatoires. Le pourcentage en poids de motifs siliconés greffés selon la septième formule dans le copolymère peut aller de 0,5 à 30 % en poids.

Selon un mode de réalisation, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon un mode de réalisation de l'invention, les polyamides à base de siloxanes peuvent notamment être :

- les polyamides selon la troisième formule précédemment exposée où m est de 15 à 50 ;
- les mélanges de deux ou plusieurs polyamides dans lesquels au moins un polyamide a une valeur de m dans la gamme de 15 à 50 et au moins un polyamide a une valeur de m dans la gamme de 30 à 50 ; des polymères selon la cinquième formule décrite précédemment avec $m_1$ choisi dans la gamme de 15 à 50 et $m_2$ choisi dans la gamme de 30 à 500 avec la partie correspondant à $m_1$ représentant 1 à 99 % en poids du poids total du polyamide et la partie correspondant à $m_2$ représentant 1 à 99 % en poids du poids total du polyamide ;
- des mélanges de polyamide selon la troisième formule décrite précédemment combinant :

    1) 80 à 99 % en poids d'un polyamide où n est égal à 2 à 10, en particulier 3 à 6, et
    2) 1 à 20 % d'un polyamide où n est dans la gamme de 5 à 500, en particulier de 6 à 100 ;

- des polyamides répondant à la sixième formule précédemment exposée où au moins l'un des Y et $Y^1$ contient au moins un substituant hydroxyle.
- des polyamides selon la troisième formule synthétisés avec au moins une partie d'un diacide activé (chlorure, dianhydride ou diester de diacide) au lieu du diacide ;
- des polyamides selon la troisième formule où X représente - $(CH_2)_3$- ou $-(CH_2)_{10}$; et
- des polyamides selon la troisième formule où les polyamides sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Selon un mode de réalisation de l'invention, les extrémités des chaînes du polymère peuvent être terminées par :

- un groupe ester d'alkyle en $C_1$ à $C_{50}$ en introduisant en cours de synthèse un monoalcool en $C_1$ en $C_{50}$,
- un groupe amide d'alkyle en $C_1$ à $C_{50}$ en prenant comme stoppeur un monoacide si la silicone est alpha , omega -diaminée, ou une monoamine si la silicone est alpha, omega -diacide carboxylique.

Selon une autre variante de réalisation de l'invention, on peut utiliser un copolymère de polyamide silicone et de polyamide hydrocarboné, soit un copolymère comportant des motifs selon la troisième ou la quatrième formule et des motifs polyamide hydrocarboné. Dans ce cas, les motifs polyamide-silicone peuvent être disposés aux extrémités du polyamide hydrocarboné.

Des agents gélifiants à base de polyamide contenant des silicones peuvent être produits par amidation silylique de polyamides à base de dimère d'acide gras. Cette approche implique la réaction de sites acides libres existant sur un polyamide comme sites terminaux, avec des oligosiloxanes-monoamines et/ou des oligosiloxanes-diamines (réaction d'amidation), ou alternativement avec des oligosiloxanes alcools ou des oligosiloxanes diols (réaction d'estérification).

La réaction d'estérification nécessite la présence de catalyseurs acides, comme il est connu dans la technique. Il est souhaitable que le polyamide ayant des sites acides libres, utilisés pour la réaction d'amidation ou d'estérification, ait un nombre relativement élevé de terminaisons acides (par exemple des polyamides ayant des indices d'acide élevés, par exemple de 15 à 20).

Pour l'amidation des sites acides libres des polyamides hydrocarbonés, des siloxanes diamines avec 1 à 300, plus particulièrement 2 à 50, et mieux encore 2, 6, 9, 5, 12, 13,5, 23 ou 31 groupes siloxanes, peuvent être utilisés pour la réaction avec des polyamides hydrocarbonés à base de dimères d'acide gras. On préfère des siloxanes diamines ayant 13,5 groupes siloxanes et les meilleurs résultats sont obtenus avec la siloxane-diamine ayant 13,5 groupes siloxane et des polyamides contenant des indices élevés de groupes terminaux acides carboxyliques.

Les réactions peuvent être effectuées dans le xylène pour extraire l'eau produite de la solution par distillation azéotropique, ou à des températures plus élevées (autour de 180 à 200 °C) sans solvant. Typiquement, l'efficacité de l'amidation et les taux de réaction diminuent lorsque le siloxane diamine est plus long, c'est-à-dire lorsque le nombre de groupes siloxanes est plus élevé. Des sites amines libres peuvent être bloqués après la réaction d'amidation initiale des diaminosiloxanes en les faisant réagir avec soit un siloxane acide, soit un acide organique tel que l'acide benzoïque.

Pour l'estérification des sites acides libres sur les polyamides, ceci peut être réalisé dans le xylène bouillant avec environ 1 % en poids, par rapport au poids total des réactifs, d'acide paratoluènesulfonique comme catalyseur.

Ces réactions effectuées sur les groupes acides carboxyliques terminaux du polyamide conduisent à l'incorporation de motifs silicone seulement aux extrémités de la chaîne de polymère.

A titre d'exemple de polymère gélifiant de type polyamide siliconé convenant à la mise en oeuvre de l'invention, on peut mentionner le copolymère bloc polyamide/polydiméthylsiloxane par exemple vendu sous la référence DC2-8178 GELLANT par la société DOW CORNING.

*Mono ou polyalkylesters de polysaccharide*

Parmi les mono ou polyalkylesters de saccharide ou de polysaccharide convenant à la mise en oeuvre de l'invention, on peut mentionner les alkyles ou polyalkylesters de dextrine ou d'inuline.

Il peut s'agir notamment d'un mono-ou poly-ester de dextrine et d'au moins un acide gras et notamment répondant à la formule suivante :

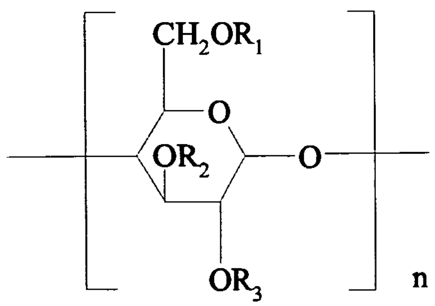

dans laquelle :

- n est un entier allant de 3 à 200, notamment allant de 20 à 150, et en particulier allant de 25 à 50,
- les radicaux $R_1$, $R_2$ et $R_3$, identiques ou différents, sont choisis parmi l'hydrogène ou un groupement acyle (R-CO-) dans lequel le radical R est un groupement hydrocarboné, linéaire ou ramifié, saturé ou insaturé, possédant de 7 à 29, en particulier de 7 à 21, notamment de 11 à 19, plus particulièrement de 13 à 17, voire 15, atomes de carbone, sous réserve qu'au moins un desdits radicaux $R_1$, $R_2$ ou $R_3$ est différent de l'hydrogène.

En particulier, $R_1$, $R_2$ et $R_3$ peuvent représenter l'hydrogène ou un groupement acyle (R-CO-) dans lequel R est un radical hydrocarboné tel que défini précédemment, sous réserve qu'au moins deux desdits radicaux $R_1$, $R_2$ ou $R_3$ sont identiques et différents de l'hydrogène.

L'ensemble des radicaux $R_1$, $R_2$ et $R_3$ peut figurer un groupement acyle (R-CO) identique ou différent, et notamment identique.

En particulier, n précédemment exposé varie avantageusement de 25 à 50, notamment est égal à 38 dans la formule générale de l'ester de saccharide utilisable dans la présente invention.

Notamment lorsque les radicaux $R_1$, $R_2$ et/ou $R_3$, identiques ou différents figurent un groupement acyle (R-CO), ceux-ci peuvent être choisis parmi les radicaux caprylique, caprique, laurique, myristique, palmitique, stéarique,

arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, et leurs mélanges.

De préférence, on utilise à titre d'ester de dextrine et d'acide(s) gras au moins un palmitate de dextrine. Celuici peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000

Des esters de dextrine, en particulier des palmitates de dextrine, sont disponibles commercialement sous la dénomination RHEOPEARL TL ou RHEOPEARL KL de la société CHIBA FLOUR.

*Copolymères di-bloc, tri-bloc, multi-bloc, radial-bloc ou en étoile*

Les polymères bloc convenant à la mise en oeuvre de l'invention sont notamment ceux décrits dans les brevets US 5,756,082 et EP 0 497 144, ainsi que dans la demande WO 98/422,98.

Egalement, les polymères bloc utilisables dans la présente invention peuvent être choisis parmi :

- les copolymères bloc (di- ou tri-bloc), tels que les polystyrènes silicones, ou les polyéthylènes silicones, décrites dans les brevets US 6,225,390, US 6,160,054, US 6,174,968 et US 6,225,390,
- les copolymères bloc ou greffés comprenant une séquence siliconée et une autre séquence ou greffon de type polyvinyle ou polyméthacrylique, tels que ceux décrits dans les brevets US 5,468,477 et US 5,725,882,
- les polymères ou copolymères résultant de la polymérisation ou copolymérisation d'un monomère éthylénique, comprenant une ou plusieurs liaisons éthyléniques, optionnellement conjuguées (ou diènes),
- les polymères ou copolymères résultant de la polymérisation ou copolymérisation d'un monomère éthylénique, notamment un copolymère de type vinyle, acrylique ou méthacrylique, qui peut être un copolymère bloc, tel qu'un copolymère di-bloc ou tri-bloc, ou même multi-bloc ou un copolymère radial ou étoilé.

L'agent gélifiant de type éthylénique peut comprendre, par exemple, un bloc styrène, un bloc alkyle-styrène, un bloc éthylène/butylène, un bloc éthylène/propylène, un bloc butadiène, un bloc isoprène, un bloc acrylate, un bloc méthacrylate, ou une combinaison de ces blocs.

Selon un mode de réalisation, les copolymères di-bloc, tri-bloc, multi-bloc et/ou radial ou étoilé peuvent comporter au moins deux segments incompatibles du point de vue thermodynamique.

Un copolymère di-bloc est habituellement défini comme étant de type A-B ou comme une séquence dans laquelle un segment dur (A) est suivi par un segment souple (B).

Un copolymère tri-bloc est habituellement défini comme étant de type A-B-A ou comme un rapport d'un segment dur, d'un segment souple et d'un segment dur.

Un copolymère multi-bloc ou radial ou étoilé peut comporter n'importe quel type de combinaison de segments durs et de segments souples, sous réserve que les caractéristiques des segments durs et des segments souples soient conservées.

A titre d'exemple de segments durs de copolymère bloc, il peut être fait mention du styrène, et à titre d'exemple de segments souples de copolymère bloc, il peut être fait mention de l'éthylène, du propylène, du butylène, et d'une combinaison de ceux-ci.

Les copolymères tri-bloc, et notamment ceux de type polystyrène/polyisoprène ou polystyrène/polybutadiène, convenant à la mise en oeuvre de l'invention peuvent être ceux commercialisés sous la référence LUVITOL HSB par la société BASF. Il peut également être fait mention des copolymères tri-bloc de type polystyrène/copoly(éthylènepropylène) ou polystyrène/ copoly(éthylène -butylène), tels que ceux commercialisés sous la référence KRATON par la société SHELL CHEMICAL CO, ou sous la référence GELLED PERMETHYL 99 A par la société PENRECO.

A titre d'exemple, encore, de copolymères bloc susceptibles de convenir à la mise en oeuvre de la présente invention, il peut également être fait mention des copolymères blocs commercialisés sous la référence VERSAGEL par la société PENRECO, ceux commercialisés sous la référence KRATON par la société SHELL et ceux commercialisés sous la référence GEL BASE par la société BROOKS INDUSTRIES.

Parmi les polymères gélifiants de phase huileuse convenant à la mise en oeuvre de l'invention, on peut notamment citer le copolymère d'éthylènediamine/stéaryle dimerdilinoléate, le polymère semi-cristallin de type poly-$C_{10-30}$ alkylacrylate, et leurs mélanges.

Selon une variante de réalisation de la présente invention, les particules huileuses calibrées structurées peuvent

contenir, notamment, en tant qu'huile, du lauroylesarcosinate d'isopropyle, et en tant que polymère gélifiant, un copolymère de dimerdilinoleate d'éthylènediamine/stéaryle.

Selon une autre variante de réalisation, les particules huileuses calibrées structurées peuvent comprendre en tant qu'huile, du squalane et en tant que polymère gélifiant, un polymère semi-cristallin de type poly-$C_{10-30}$ alkylacrylate.

Selon encore un autre mode de réalisation, les particules huileuses calibrées structurées peuvent comprendre en tant que phase huileuse un mélange de laurylsarcosinate d'isopropyle et d'isononanoate d'isononyle et en tant que polymère gélifiant le copolymère dimerdilinoleate d'éthylènediamine/stéaryle.

Selon encore un autre mode de réalisation, les particules huileuses calibrées structurées conformes à la présente invention peuvent comprendre en tant que phase huileuse un mélange d'isononanoate d'isononyle et d'octyldodécanol et en tant que polymère gélifiant, le copolymère dimerdilinoleate d'éthylènediamine/stéaryle.

En outre, les particules peuvent comprendre un autre gélifiant de type dérivé d'acide alcoyle glutamique, par exemple le dibutylamide d'acide laurylglutamique, commercialisé par la société AJINOMOTO sous le nom "GELLING AGENT GP-1".

## SUBSTANCES ACTIVES

Les particules selon l'invention peuvent comprendre, en outre, au moins une substance active.

Les substances actives incorporées dans les particules conformes à l'invention sont avantageusement lipophiles. Elles peuvent notamment être choisies parmi les substances convenant pour le maquillage et/ou le soin de la peau, y compris, par exemple, le cuir chevelu et les lèvres.

Par l'expression "actif" ou "substance active", on entend, dans le cadre de la présente invention, un composé qui a par lui-même, c'est-à-dire ne nécessitant pas l'intervention d'un agent extérieur pour l'activer, une activité biologique.

L'activité biologique de l'actif peut être en particulier utile pour le soin des peaux grasses, qui peut être en particulier :

- une activité desquamante, et/ou
- une activité anti-microbienne (notamment sur P. acnes), et/ou
- une activité anti-inflammatoire, et/ou
- une activité sébo-régulatrice, et/ou
- une activité anti-oxydante.

A ce titre, l'actif peut donc être choisi parmi : les agents desquamants et/ou anti-microbiens et/ou anti-inflammatoires et/ou sébo-régulateurs et/ou anti-oxydants.

1. <u>Agents desquamants</u>

Par "agent desquamant", on entend tout composé capable d'agir :

- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol et certains dérivés d'acide jasmonique ;

- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

L'acide n-octanoyl-5-salicylique est préféré pour une utilisation dans la présente invention.

2. <u>Agents anti-microbiens</u>

Les agents anti-microbiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et

ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le pidolate de cuivre, l'acide salicylique, l'iodopropynyl butylcarbamate, le farnesol, les phytosphingosines et leurs mélanges.

Les agents antimicrobiens préférés sont l'octoxyglycérine, le pidolate de cuivre, l'acide salicylique et l'iodopropynyl butylcarbamate.

3. Agents anti-inflammatoires

Comme agents anti-inflammatoires ou apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3-stéaroyloxy glycyrrhetique), l'acide ursolique et ses sels, l'acide oléanolique et ses sels, l'acide bétulinique et ses sels, le bisabolol, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'Echium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, les extraits de Centella asiatica, le piperonal, un extrait de clou de girofle, un extrait d'Epilobium Angustifolium, l'aloe vera, un extrait de Bacopa monieri, les phytostérols, la niacinamide, la cortisone, l'hydrocortisone, l'indométhacine et la beta méthasone.

Les agents anti-inflammatoires préférés pour une utilisation dans la présente invention sont les extraits de Centella asiatica, l'acide β-glycyrrhétinique et ses sels, l'alpha-bisabolol et la niacinamide.

4. Agents sébo-régulateurs

Lorsque la composition selon l'invention comprend un agent sébo-régulateur tel qu'un inhibiteur de 5α-réductase, celui-ci peut notamment être choisi parmi :

- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5® ;
- un extrait de Laminaria saccharina commercialisé notamment par la société SECMA sous la dénomination commerciale Phlorogine® ;
- un extrait de Spiraea ulmaria commercialisé notamment par la société SILAB sous la dénomination commerciale Sebonormine® ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED ;
- des extraits de plantes du genre Silybum ;
- des extraits végétaux contenant des sapogénines et en particulier les extraits de Dioscorées riches en diosgénine ; et
- des extraits d'Eugenia caryophyllata contenant de l'eugénol et du glucoside d'eugényle.

Les sels de zinc sont préférés pour une utilisation dans la présente invention.

5. Agents anti-oxydants

Les agents anti-oxydants préférés pour une utilisation dans la présente invention peuvent être choisis parmi

le tocophérol et ses esters, tels que l'acétate de tocophérol ; le BHT et le BHA.

Tous ces actifs sont particulièrement utiles, comme actif visant le follicule sébacé.

D'autres actifs peuvent aussi être utilisés dans le cadre de la présente invention, au titre d'actifs visant plus spécifiquement le follicule pileux.

A titre d'exemple de ces actifs, il peut être fait mention :

- des agents inhibiteurs de la chute du cheveu ainsi que les agents stimulateurs de la pousse du cheveu tels le minoxidil, la biotine, le fmastèride, le 2,4 dipirymidine N-oxyde, le panthénol et leurs dérivés, la flavanone T, ou plus généralement tout extrait végétal, possédant une activité anti 5-α-réductase de type I ou II,
- des agents inhibiteurs de la pousse du cheveu ou du poil tels que les sérines protéases décrites dans le brevet US 6 407 056, l'acide caféique, la quercétine, le gallate de propyle, l'acide norhydroguaiarétique ou NDGA, l'indométhacine, l'hydrochlorure d'eflornithine, les extraits végétaux tels décrits dans le brevet US 6 171 595 comme les extraits de clou de girofle, de bouton de rose, de pimprenelle (burnet), de gambir, les composés décrits dans le brevet US 6 075 052, le tétramisole, l'orthovanadate de sodium, le levamisole, le chromoglycate disodique, le nitrate de vanadium et le nitrate de gallium tel que décrit dans le brevet US 6 020 006, ainsi que les composés décrits dans les brevets US 4 885 289, US 4 720 489, US 5 132 293, US 5 096 911, US 5 095 007, US 5 143 925, US 5 328 686, US 5 440 090, US 5 364 885, US 5 411 991, US 5 648 394, US 5 468 476, US 5 475 763, US 5 455 608, US 5 674 477, US 5 728 736 et 5 652 273 et dans les demandes de brevet WO 94/27586, WO 94/27563 et WO 98/03149. On peut également utiliser les extraits de genévrier tels que décrits dans le brevet US 6 375 948,
- des agents anti-pelliculaires tels que la pyrithione de zinc, et
- leurs mélanges.

A titre d'exemple de substance active convenant plus particulièrement à la mise en oeuvre de l'invention, on peut mentionner l'acide n-octanoyl-5 salicyclique, l'α-bisabolol, l'α-tocophérol, le butylcarbamate d'iodopropynyle, l'acide ursolique, et leurs mélanges.

La teneur en substance(s) ou molécule(s) active(s) présente(s) dans les particules conformes à l'invention peut varier de 0,05 à 70 % en poids de 0,1 à 50 % en poids et en particulier de 0,5 à 40 % en poids par rapport au poids total de la particule.

## AGENTS TENSIOACTIFS

Les particules selon l'invention peuvent en outre comprendre au moins un tensioactif.

La présence de tensioactif(s) et leur nature chimique sont généralement déterminés par la nature du procédé de préparation desdites particules.

Ainsi, lorsque les particules sont préparées selon le procédé décrit ci-après, impliquant une étape d'émulsification, il est introduit dans le mélange phase huileuse-phase aqueuse, au moins un agent tensioactif choisi parmi les agents tensioactifs non-ioniques, les agents tensioactifs ioniques, et leur mélanges.

Les tensioactifs non-ioniques, ou leurs mélanges, avantageusement utilisés dans le cadre de la présente invention sont des tensioactifs, ou leurs mélanges, ayant une HLB supérieur à 5.

A titre d'exemple de tensioactifs non-ioniques convenant pour la mise en oeuvre de l'invention, on peut citer les :

- mono- ou polyalkylesters ou éthers de glycérol oxyethyléné, ou non, tels que ceux décrits dans le brevet US 6 541 018 ;
- mono- ou polyalkylesters ou éthers de sorbitan oxyéthyléné, ou non, tels que ceux décrits dans US 6 335 022 ;
- mono- ou polyalkylesters ou éthers de polyoxyde d'éthylène, tels que ceux décrits dans US 6 375 960;
- mono- ou polyalkylesters ou éthers de sucre oxyethyléné, ou non, tels que ceux décrits dans US 6 689 371 ; et
- leurs mélanges.

Les tensioactifs ioniques utilisables dans le cadre de la présente invention peuvent être de type anionique, de type cationique ou de type amphiphile.

Les tensioactifs anioniques peuvent être, notamment, choisis parmi :

- les alkenyl succinate alkoxylés tels que ceux cités dans le brevet US 6 461 625 ;
- les alkyl éther citrates tels que ceux cités dans le brevet US 6 413 527 ;
- les alkyl ester phosphoriques tels que ceux décrits dans le brevet US 6 274 150 ; et
- leurs mélanges.

Les chaînes alkyles des tensioactifs anioniques convenant à la mise en oeuvre de l'invention sont avantageusement comprises dans l'intervalle allant de $C_{12}$ à $C_{24}$, et peuvent être saturées ou non et/ou linéaires ou ramifiées.

- Les tensioactifs anioniques utilisables pour la présente invention peuvent être également des lipoaminoacides, des dérivés alkylsulfoniques, et leurs mélanges.

Les lipoaminoacides peuvent être notamment choisis parmi les acylglutamates mono- et di-sodiques comme le sel disodique de l'acide N-stéaroyl L-glutamique commercialisé sous la dénomination ACYLGLUTAMATE HS21 par la société AJINOMOTO.
Les dérivés alkylsulfoniques peuvent être notamment choisis parmi les dérivés alkylsulfoniques de première formule suivante :

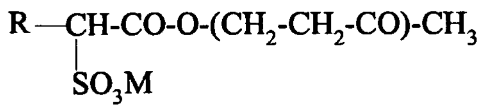

dans laquelle R représente un radical alkyle comportant de 16 à 22 atomes de carbone, et notamment un radical en $C_{16}H_{33}$ et $C_{18}H_{37}$ pris en mélange ou séparément, et M est un métal alcalin tel que le sodium, par exemple.

- Les tensioactifs cationiques convenant pour la préparation des particules et/ou dispersions conformes à l'invention peuvent être notamment choisis parmi les sels d'ammonium quaternaires, les amines grasses et leurs sels, et leurs mélanges.

Les sels d'ammonium quaternaires sont par exemple :

a) ceux qui présentent la seconde formule générale suivante :

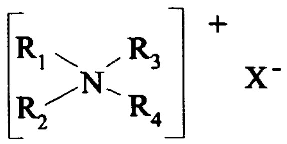

dans laquelle les radicaux $R_1$ à $R_4$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène ($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone ; X est un anion choisi dans le groupe des halogénures, phosphates, acétates, lactates, alkyl($C_2$-$C_6$)sulfates, alkyl-ou-alkylarylsulfonates.
Comme sels d'ammonium quaternaire présentant la seconde formule précédemment exposée, avantageusement utilisée, on peut mentionner d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthylammonium, de benzyl diméthyl stéaryl-ammonium ou encore, d'autre part, le chlorure de stéaramidopropyldiméthyl (myristyl acétate) ammonium commercialisé sous la dénomination "CERAPHYL 70" par la société VAN DYK.
b) les sels d'ammonium quaternaire de l'imidazolinium, comme par exemple ceux de troisième formule générale suivante :

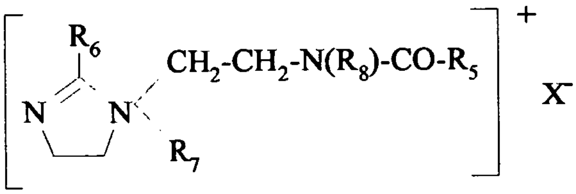

dans laquelle $R_5$ représente un radical alcényle ou alkyle comportant de 8 à 30 atomes de carbone, par exemple dérivé des acides gras du suif ; $R_6$ représente un atome d'hydrogène, un radical alkyle comportant de 1 à 4 atomes de carbone ou un radical alcényle ou alkyle comportant de 8 à 30 atomes de carbone ; $R_7$ représente un radical alkyle comportant de 1 à 4 atomes de carbone ; $R_8$ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates.

En particulier, $R_5$ et $R_6$ désignent un mélange de radicaux alcényle ou alkyle comportant de 12 à 21 atomes de carbone, par exemple dérivés des acides gras du suif, $R_7$ désigne un radical méthyle, $R_8$ désigne l'hydrogène. Un tel produit est par exemple vendu sous la dénomination "REWOQUAT W 75" par la société REWO.

c) les sels de diammonium quaternaire de quatrième formule générale suivante

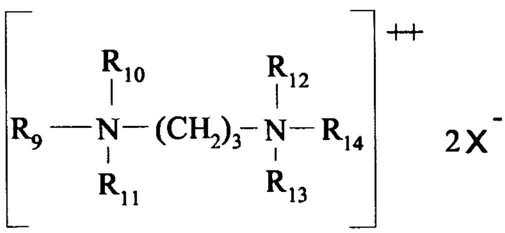

dans laquelle $R_9$ désigne un radical aliphatique comportant environ de 16 à 30 atomes de carbone ; $R_{10}$, $R_{11}$, $R_{12}$, $R_{13}$, et $R_{14}$ sont choisis parmi l'hydrogène ou un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium.

A titre d'exemple d'agents tensioactifs convenant à la mise en oeuvre de l'invention, on peut citer le PEG-30 stéarate de glycéryle, le stéaroylglutamate de disodium, et leurs mélanges.

Selon encore une autre variante de réalisation, les dispersions conformes à la présente invention peuvent comprendre en tant que tensioactif non-ionique, un mélange de PEG-30 stéarate de glyceryle et de stéaroylglutamate de disodium.

La teneur en tensioactif non ionique et/ou tensioactif ionique, utilisés pour la préparation des particules et/ou des dispersions conformes à l'invention peut avantageusement varier de 0,5 et 50 % en poids, voire de 1 à 40 % en poids, et en particulier de 5 à 20 % en poids par rapport au poids total de la dispersion.

## POLYMÈRES ÉMULSIONNANTS

Les particules peuvent également contenir un polymère émulsionnant, c'est-à-dire un polymère amphiphile.

Parmi les polymères émulsionnants convenant à la mise en oeuvre de l'invention, il peut être fait mention :

- des copolymères di et tribloc POE-POP tels que ceux décrits dans le brevet US 6 464 990 ;
- des tensioactif siliconé et polyoxyéthyléné tels que ceux décrits dans le brevet US 6 120 778 ;
- des AMPS hydrophobés non réticulés tels que ceux décrits dans EP 1 466 588 ;
- des polymères acryliques amphiphiles, tels que les PEMULEN TR-1 ou TR-2 ou équivalent ;
- des polymères associatifs et gélifiants décrits dans US 2003/0138465 ;
- des polymères thermogélifiants tels que ceux décrits dans les demandes US 2004/0214913, US 2003/0147832 et US 2002/0198328 et FR 2 856 923.

Lorsqu'ils sont présents, le ou les polymères émulsionnants peuvent être introduits en une teneur variant de 0,1 à 15 % en poids, voire de 0,1 % à 10 % en poids, et plus en particulier de 0,1 à 5 % en poids par rapport au poids

total de la dispersion.

**PROCEDE D'OBTENTION DES DISPERSIONS**

Les particules huileuses calibrées et sphériques, structurées, conformes à l'invention, peuvent être obtenues sous la forme d'une dispersion au moyen d'un procédé comprenant au moins les étapes consistant à :

- émulsifier un mélange d'au moins une huile ou une phase huileuse et d'au moins un polymère gélifiant de la phase huileuse avec une phase aqueuse et/ou hydrosoluble à une température supérieure à la température de gélification du polymère,
- soumettre le mélange à un processus conduisant à l'obtention des particules huileuses, à une température d'au moins 5 à 10 °C supérieure à la température de fusion du mélange mis en oeuvre dans l'étape précédente, et
- refroidir la dispersion de particules ainsi obtenue.

On précise que la présence d'eau dans la première étape du procédé et la réalisation de la deuxième étape à chaud sont des conditions cumulatives nécessaires à l'obtention de particules calibrées sphériques selon l'invention.

La mesure de la viscosité est bien effectuée au niveau du mélange initial, à savoir du "macrogel", et non lorsque les particules sont déjà formées, comme cela a déjà été précisé plus haut.

Le procédé selon l'invention peut, le cas échéant, comprendre en outre une étape consistant à diluer la phase continue du mélange avant l'étape de refroidissement.

Au sens de la présente invention, on entend désigner par "processus conduisant à l'obtention des particules huileuses", une action de type cisaillement ou un mécanisme de type inversion de phase.

La température à laquelle est réalisée l'étape d'émulsification, est avantageusement supérieure à 40 °C, et avantageusement inférieure à 95 °C.

Ainsi, à l'issue du procédé, les dispersions conformes à l'invention comprennent dans une phase aqueuse et/ou hydrosoluble des particules huileuses calibrées comprenant une phase huileuse structurée par au moins un polymère gélifiant.

La nature du processus exercé sur le mélange phase huileuse/polymère gélifiant détermine la taille des particules à obtenir.

Ainsi, pour les particules submicroniques, d'une taille moyenne d'environ 150 nm à 1 µm, on peut utiliser avantageusement des procédés développant un cisaillement turbulent tels que les ultrasons, l'homogénéisation à haute pression (pression d'usage entre 50 et 1000 bar) par exemple en utilisant le SOAVI OBL 20® de NIRO SOAVI, ou le MICROFLUIDIZER® de MICROFLUIDICS. On peut également utiliser les procédés ne nécessitant pas d'apport d'énergie mécanique tels que ceux mettant enjeu une inversion de phase lors de l'émulsification, comme la P.I.T (phase inversion temperature), l'inversion de composition (par exemple par ajout d'un tensio actif hydrophile dans une émulsion E/H pour l'inverser en H/E), et la nanoprécipitation, telle que décrite dans le brevet EP 0 447 318.

Pour les particules microniques, d'une taille moyenne d'environ 1 µm à 15 µm, on peut utiliser, par exemple, des procédés permettant d'obtenir la plus faible polydispersité possible tels que le cisaillement contrôlé d'émulsions viscoélastiques comme décrit dans la demande FR 2 747 321 et le brevet US 5 558 820, des procédés en continu tels que décrits dans le brevet US 5 688 842 et la demande WO 02/40574 ou ceux, plus généralement utilisant un moulin colloïdal, un mélangeur statique, un micromélangeur, une pale cadre, ou encore une membrane poreuse comme décrit dans le brevet US 5 326 484. On peut également utiliser des procédés faisant appel au contrôle du mûrissement (US 6 160 061), au gonflement d'un latex "templating agent" (EP 719 087), aux instabilités de Rayleigh (Weitz, Langmuir, 16, 347-351, (2000)) ou par fractionnement d'émulsions polydisperses (Bibette, J. Coll. Int. Sci., vol 147, n°2, 474-478, (1991)).

Afin de faciliter la formation des particules, lors de l'étape d'émulsification, on peut, par exemple, utiliser un ou des tensioactifs non-ioniques ou ioniques et/ou des polymères émulsionnants hydrophobes, tels que définis précédemment.

Par ailleurs, dans le cas par exemple de procédés développant un cisaillement laminaire pour obtenir une distribution de taille de particules homogène, il peut être, éventuellement, avantageux d'ajuster le rapport entre la viscosité de la phase huileuse dispersée et la viscosité de la phase aqueuse et/ou hydrophile continue dans un rapport allant de 0,01 à 5, voire de 0,05 à 2. Cet ajustement peut se faire, notamment, par l'addition de tensioactifs et/ou de polymère émulsionnant tels que ceux décrit précédemment et/ou de polymères gélifiants hydrophiles.

Ainsi, selon un mode de réalisation de l'invention, la phase continue aqueuse et/ou hydrosoluble peut, en outre, comprendre au moins un polymère hydrophile gélifiant.

Avantageusement, lorsqu'il est présent, le polymère hydrophile gélifiant est introduit dans la phase continue aqueuse, ou hydrophile, en une proportion allant de 0,1 % à 30 % en poids, notamment de 0,05 % à 15 % en poids par rapport au poids total de la composition.

A titre d'exemple de polymères hydrophiles gélifiants, il peut être fait mention, notamment, des carbomers, de l'acrylamidométhyle propane sulfonique (AMPS), des dérivés de cellulose ou de guar. A titre de dérivés de guar susceptibles d'être avantageusement utilisés dans la mise en oeuvre de la présente invention, il est possible de citer l'hydroxypropylguar commercialisé sous la référence JAGUAR HP® 105 par la société RHODIA.
Ainsi, le mélange phase huileuse — phase aqueuse et/ou hydrosoluble peut comprendre, en outre, un composé choisi parmi un agent tensioactif, un polymère émulsionnant, un polymère gélifiant hydrophile, et leurs mélanges, et de préférence un mélange d'un agent tensioactif, d'un polymère émulsionnant et d'un polymère gélifiant hydrophile.
Selon une variante, le mélange phase huileuse — phase aqueuse peut comprendre en outre au moins une substance active. Cette substance active peut être telle que définie précédemment.
Ainsi, en raison du procédé de préparation des particules conformes à l'invention, ces dernières sont avantageusement dépourvues de solvant volatile.

## DISPERSION

Conformément au procédé d'obtention des particules conformes à l'invention, tel que décrit précédemment, ces dernières sont obtenues en dispersion dans une phase continue et/ou hydrosoluble.
La teneur en particules huileuses calibrées, comprenant une phase huileuse structurée par un polymère gélifiant, selon l'invention présentes dans la phase continue aqueuse et/ou hydrosoluble peut notamment être telle que la fraction massique huileuse dispersée dans la phase aqueuse et/ou hydrosoluble peut varier de 5 à 89 % en poids, notamment de 20 à 85 % en poids, voire de 40 à 80 % en poids, et en particulier de 60 à 80 % en poids par rapport au poids total de la dispersion.
Les particules huileuses calibrées, structurées, conformes à l'invention, avantageusement ne s'agrègent pas dans la dispersion dans laquelle elles sont obtenues, et leurs spécificités granulométriques en terme de taille et d'indice de distribution y sont avantageusement conservées.
La phase continue aqueuse et/ou hydrosoluble convenant à la mise en oeuvre de l'invention peut être, avantageusement, de l'eau et/ou un solvant organique hydrosoluble, comme par exemple des glycols comme la glycérine, le dipropylène glycol, seuls ou en mélanges.
On entend désigner par l'expression "solvant hydrosoluble", au sens de la présente invention, un composé liquide à température ambiante et miscible à l'eau (miscibilité dans l'eau supérieure à 50 % en poids à 25 °C, et à pression atmosphérique).
Parmi les solvants hydrosolubles pouvant être utilisés dans les dispersions conformes à l'invention, on peut mentionner, notamment les monoalcools inférieurs ayant de 1 à 5 atomes de carbone, tels que l'éthanol et l'isopropanol, les glycols ayant de 2 à 8 atomes de carbone, tels que l'éthylène glycol, le propylène glycol, le 1,3-butylèneglycol et le dipropylèneglycol, les cétones en $C_3$ et $C_4$, la glycérine et les aldéhydes en $C_2$-$C_4$.
Selon encore une autre variante de réalisation, les dispersions conformes à la présente invention peuvent comprendre en tant que phase aqueuse continue, de l'eau déminéralisée.
Selon encore une autre variante de réalisation, les dispersions conformes à l'invention peuvent comprendre en tant que tensioactif non-ionique un mélange de PEG-30 stéarate de glyceryle et de stéaroylglutamate de disodium, et en tant que polymère hydrophile gélifiant, de l'hydroxypropyle guar.

## COMPOSITION COSMETIQUE OU DERMATOLOGIQUE

Les particules et/ou les dispersions conformes à l'invention, comprenant une substance active peuvent permettre l'administration spécifique de substances cosmétiquement et/ou dermatologiquement actives au niveau de la glande sébacée et/ou du follicule pileux. Ces particules et/ou dispersions peuvent être, avantageusement, introduites dans différentes formulations cosmétiques et/ou dermatologiques destinées à une application topique sur la peau ou les cheveux.
Ainsi, les particules et/ou les dispersions conformes à la présente invention peuvent être utilisées à titre de véhicule pour au moins une substance active pour la préparation de composition(s) cosmétique(s) utilisable dans le domaine du maquillage et/ou du soin de la peau, pour promouvoir la pénétration, au moins partiellement de la substance active, dans les glandes sébacées et/ou follicules pileux.
Ainsi, la présente invention a encore pour objet des compositions cosmétiques ou dermatologiques comprenant au moins des particules et/ou au moins une dispersion telle que précédemment définies.
Les compositions comportant des particules et/ou des dispersions conformes à l'invention peuvent être des compositions pour le soin, l'hygiène et/ou le maquillage de la peau ou des phanères.
En l'occurrence, une composition selon l'invention peut se présenter sous forme de mascara, de produit pour les sourcils, d'eyeliner, de fard à paupières, de fard à joues, de fond de teint, de produit pour les lèvres, de produit de maquillage du corps, de produit de maquillage ou de soin des cheveux.

Elles peuvent être, par exemple, destinées à un usage capillaire et être en particulier des shampooings, des après-shampooings, des lotions capillaires, en particulier de soin capillaire.
Avantageusement, la composition peut contenir de 0,01 à 40 % en poids, notamment de 0,1 à 25 % en poids voire de 0,2 à 20 % en poids, de particules conformes à la présente invention par rapport au poids total de la composition.
La composition cosmétique ou dermatologique peut se présenter sous la forme de lotion, d'émulsion huile-dans-eau (H/E) ou eau-dans-huile (E/H) ou multiple (E/H/E), de gel aqueux ou hydroalcoolique, de crème, lait...

## ADDITIFS

Les compositions cosmétiques conformes à l'invention peuvent également comporter tout additif usuellement utilisé dans le domaine concerné sous réserve que ces additifs n'altèrent pas la propriété des compositions à pénétrer dans les glandes sébacées et/ou les follicules pileux.
Dans le cadre des compositions destinées notamment au maquillage, les additifs susceptibles de convenir à la mise en oeuvre de l'invention peuvent être choisis notamment parmi des matières colorantes, comme les nacres et les pigments, des charges, des antioxydants, des agents filmogènes, et le cas échéant des auxiliaires de filmification, des huiles essentielles, des conservateurs, des parfums, des agents hydratants, des agents antiseptiques, des neutralisants, et leurs mélanges.
Bien entendu l'homme du métier veillera également à choisir les éventuels additifs complémentaires et/ou leur quantité de telle manière que, les propriétés avantageuses de la composition selon l'invention, ne soient pas ou substantiellement pas, altérées par l'adjonction envisagée.
La présente invention a encore pour objet un procédé cosmétique de maquillage et/ou de soin non thérapeutique, comprenant au moins l'étape d'application sur la peau d'une composition telle que définie précédemment.
Les exemples de dispersions de particules et de compositions présentés ci-après sont donnés à titre illustratif et sans caractère limitatif de l'invention.

## EXEMPLES

**Exemple 1 :** Microparticules contenant un agent kératolytique

La dispersion est réalisée dans un bécher de diamètre interne 72 mm comportant une double enveloppe permettant de réguler sa température par l'intermédiaire d'un bain chauffant. On y introduit une solution aqueuse composée de 12,8 g de PEG-30 stéarate de glyceryle (TAGAT S® de la Société DEGUSSA), 3,2 g de stéaroylglutamate de disodium (AMISOFT HS21P® de la Société AJINOMOTO) et 64g d'eau déminéralisée. Le mélange est porté à 85 °C.
Par ailleurs, on réalise le mélange suivant : 30 g de copolymère de dimerdilinoleate d'éthylenediamine/stéaryle (UNICLEAR 100 VG® de la Société ARIZONA CHEMICAL), 84 g de lauroylsarcosinate d'isopropyle (ELDEW SL-205® de la Société AJINOMOTO) et 6 g d'acide n-octanoyl-5 salicylique que l'on porte à une température de 85 °C.
Une fois homogène, celui-ci est introduit en 10 minutes dans la solution aqueuse de tensioactif maintenue sous agitation par une pale de largeur 70 mm et d'épaisseur 2 mm avec une vitesse de rotation de 160 rpm. Après 3 minutes, la vitesse de rotation est portée à 300 rpm et maintenue pendant 30 min.
L'émulsion est ensuite diluée avec 100g d'eau déminéralisée à 80 °C pour atteindre une fraction massique en phase dispersée de 40 %, puis refroidie à température ambiante.
On obtient une dispersion homogène et stable de microparticules avec une granulométrie de 2,47 $\mu$m (d[0,5]) et un coefficient d'uniformité (U) de 0,27.

**Exemple 2 :** Microparticules contenant de l'$\alpha$-bisabolol

La dispersion est réalisée dans un bécher de diamètre interne 72 mm comportant une double enveloppe permettant de réguler sa température par l'intermédiaire d'un bain chauffant. On y introduit une solution aqueuse composée de 6,4 g de PEG-30 stéarate de glyceryle (TAGAT S® de la Société DEGUSSA), 1,6 g de stéaroylglutamate de disodium (AMISOFT HS21P® de la Société AJINOMOTO) et 32 g d'eau déminéralisée. Le mélange est porté à 80 °C.
Par ailleurs on réalise le mélange suivant : 48 g de poly C10-30 alkyl acrylate (DORESCO IPA 13-6® de la Société LANDEC CORPORATION), 56 g de squalane et 56 g d' $\alpha$-bisabolol, que l'on porte à une température de 80 °C.
Une fois homogène, celui-ci est introduit en 10 minutes dans la solution aqueuse de tensioactif maintenue sous agitation par une pale de largeur 70 mm et d'épaisseur 2 mm avec une vitesse de rotation de 160 rpm. Après 3 minutes la vitesse de rotation est portée à 400 rpm et maintenue pendant 30 minutes.
L'émulsion est ensuite diluée avec 200 g d'eau déminéralisée à 80 °C pour atteindre une fraction massique en phase dispersée de 40 %, puis refroidie à température ambiante.
On obtient une dispersion homogène de microparticules avec une granulométrie de 4,2 $\mu$m (d[0,5]) et un

coefficient d'uniformité (U) de 0,25.

**Exemple 3 :** Microparticules contenant de la vitamine E et un agent antibactérien

La dispersion est réalisée dans un bécher de diamètre interne 72 mm comportant une double enveloppe permettant de réguler sa température par l'intermédiaire d'un bain chauffant. On y introduit une solution aqueuse composée de 1,92 g de PEG-30 stéarate de glyceryle (TAGAT S de la Société DEGUSSA), 0,48 g de stéaroylglutamate de disodium (AMISOFT HS21P de la Société AJINOMOTO), 1,2 g d' hydroxypropyl guar (JAGUAR HP105 de la Société RHODIA) et 116,4 g d'eau déminéralisée. Le mélange est porté à 80 °C.

Par ailleurs on réalise le mélange suivant : 20 g de copolymère de dimerdilinoleate d'éthylenediamine/stéaryle (UNICLEAR 100VG® de la Société ARIZONA CHEMICAL), 28,24 g de lauroylsarcosinate d'isopropyle (ELDEW SL-205® de la Société AJINOMOTO)1, 28,24 g d'isononanoate d'isononyle, 3,2 g d' $\alpha$-tocophérol et 0,32 g de butylcarbamate d'iodopropynyle (GLYCACIL® de la Société LONZA), que l'on porte à une température de 80 °C.

Une fois homogène, celui-ci est introduit en 10 minutes dans la solution aqueuse de tensioactif maintenue sous agitation par une pale de largeur 70 mm et d'épaisseur 2 mm avec une vitesse de rotation de 160 rpm. Après 3 minutes la vitesse de rotation est portée à 400 rpm et maintenue pendant 30 minutes.

L'émulsion est ensuite diluée avec 150g d'eau déminéralisée à 80 °C pour atteindre une fraction massique en phase dispersée de 23 %, puis refroidie à température ambiante.

On obtient une dispersion homogène de microparticules avec une granulométrie de 7,18 $\mu$m (d[0,5]) et un coefficient d'uniformité (U) de 0,40.

**Exemple 4** : Nanoparticules d'acide ursolique

On prépare une solution aqueuse composée de 9 g de PEG-30 stéarate de glyceryl, 1 g de stéaroylglutamate de disodium et 390 g d'eau déminéralisée. Le mélange est porté à 85 °C. Par ailleurs on réalise le mélange suivant : 30 g de copolymère de dimerdilinoleate d'éthylenediamine/stéaryle (UNICLEAR 100 VG® de la Société ARIZONA CHEMICAL), 59,5 g d'isononanoate d'isononyle, 10 g d'octyldodecanol, 0,5 g d'acide ursolique, que l'on porte à une température de 85 °C.

Celui-ci est introduit dans la solution aqueuse de tensioactif maintenue sous agitation par un dispositif de type rotor-stator (UltraTurrax T50). Après 5 minutes, le mélange est homogénéisé à 85 °C à l'aide d'un homogénéisateur SOAVI PANDA, en 2 passages à une pression d'environ 400 bar.

La dispersion est ensuite refroidie à température ambiante.

On obtient une dispersion homogène de nanoparticules avec un diamètre moyen de 250 nm.

**Exemple 5 :** Emulsion H/E contenant les microparticules de l'invention

| | |
|---|---|
| Phase A | |
| Disodium EDTA | 0,10 % |
| Glycerin | 5,00 % |
| Conservateur | 0,20 % |
| Eau Déminéralisée | 50,00 % |
| Phase B | |
| Polyglyceryl-3 Methylglucose Distearate (Tego Care 450 de la Société Degussa) | 1,50 % |
| Glyceryl Stearate SE (Tegin Vegetable Based de la Société Degussa) | 1,50 % |
| Alcool stéarique | 1,00 % |
| Stearic Acid | 2,00 % |
| Vitis Vinifera huile de pépin de raisin | 1,00 % |
| Prunus Armeniaca huile de noyau d'abricot | 2,00 % |
| Conservateur | 0,20 % |

(suite)

| | |
|---|---|
| Phase C | |
| Cyclohexasiloxane | 2,00 % |
| Diméthicone (et) Diméthiconol (DC 2-9085 de la Société Dow Corning) | 1,00 % |
| Phase D | |
| Carbomer | 0,40 % |
| Eau Déminéralisée | 15,00 % |
| Phase E | |
| Gomme de xanthane | 0,20 % |
| Eau Déminéralisée | 7,00 % |
| Phase F | |
| Triethanolamine | 0,40 % |
| Eau Déminéralisée | 2,00 % |
| Phase G | |
| Dispersion de microparticules de l'Exemple 1 | 7,50 % |

On obtient une émulsion fme et légère qui permet de lutter contre les peaux grasses.

**Exemple 6** : Emulsion H/E contenant les microparticules de l'invention

| | |
|---|---|
| Phase A | |
| PEG-20 methyl glucose sesquistearate (GLUCAMATE SSE 20 de Chemron) | 2,00 % |
| Disodium EDTA | 0,10 % |
| Glycerin | 5,00 % |
| Conservateur | 0,20 % |
| Eau déminéralisée | 44,70 % |
| Phase B | |
| Methyl glucose sesquistearate | 2,00 % |
| Alcool stéarique (et) Ceteareth-20 | 2,00 % |
| Cyclohexasiloxane | 5,85 % |
| C12-15 alkyl benzoate | 5,85 % |
| Conservateur | 0,10 % |
| Phase C | |
| Polyacrylamide (et) C13-14 isoparaffine (et) Laureth-7 (Sepigel 305 de Seppic) | 1,00 % |
| Ammonium Polyacryloyldimethyl Taurate (Hostacerin AMPS de Clariant) | 1,20 % |
| Phase D | |
| Biosaccharide Gum-1 (Fucogel 1000PP de Solabia) | 5,00 % |
| Phase E | |
| Aluminum Octenylsuccinate amidoné | 3,00 % |

(suite)

| | |
|---|---|
| Phase E | |
| Phase F | |
| Dispersion de microparticules de l'Exemple 3 | 22,00 % |

On obtient une crème douce, fraîche, adaptée au soin des peaux acnéiques.

**Exemple 7** : Microparticules contenant de l'acide b-glycyrrhétinique

La dispersion est réalisée dans un bécher de diamètre interne 72 mm comportant une double enveloppe permettant de réguler sa température par l'intermédiaire d'un bain chauffant. On y introduit une solution aqueuse composée de 6,4 g de PEG-30 stéarate de glycéryle (Tagat S® de la société DEGUSSA), de 1,6 g de stéaroylglutamate de disodium (Amisoft HS21P® de la société Ajinomoto) et 32 g d'eau déminéralisée. Le mélange est porté à 85 °C.
Par ailleurs, on réalise le mélange suivant :

- 40 g de nylon-611/diméthicone copolymère (DC2-8178 Gellant de la société DOW CORNING),
- 60 g de diméthicone (DC Fluid 200-5cs de la société DOW CORNING),
- 57 g d'isopropyl lauroylsarcosinate (Eldew SL-205 de la société AJINOMOTO), et
- 3 g d'acide b-glycyrrhétinique, que l'on porte à une température de 85 °C.

Une fois homogène, celui-ci est introduit en 10 minutes dans la solution aqueuse de tensioactif maintenue sous agitation par une pale de largeur 70 mm et d'épaisseur 2 mm avec une vitesse de rotation de 160 rpm. Après 3 minutes, la vitesse de rotation est portée à 400 rpm et maintenue pendant 30 minutes.
L'émulsion est ensuite diluée avec 200 g d'eau déminéralisée à 80 °C pour atteindre une fraction massique en phase dispersée de 40 %, puis refroidie à température ambiante.
On obtient une dispersion homogène de microparticules avec une granulométrie de 1,2 µm (d[0,5]) et un coefficient d'uniformité (U) de 0,34.

## Revendications

1. Particules huileuses calibrées et sphériques comprenant au moins une phase huileuse structurée par au moins un polymère gélifiant, **caractérisées en ce qu'**elles possèdent une taille moyenne inférieure ou égale à 15 µm, **en ce que** ladite phase huileuse structurée possède un point de fusion supérieur ou égal à 40 °C, et **en ce que** leur indice de circularité est compris entre 0,9 et 1.

2. Particules huileuses calibrées et sphériques selon la revendication 1, **caractérisées en ce que** ledit polymère gélifiant est choisi parmi un polymère semi-cristallin, un polyamide, un polyamide siliconé, un monoalkylester ou un polyalkylester de polysaccharide, un copolymère dibloc et/ou tribloc et/ou multibloc et/ou radial-bloc, et leurs mélanges.

3. Particules huileuses calibrées et sphériques selon la revendication 1, **caractérisées en ce que**, ledit polymère gélifiant est de nature et/ou à une teneur suffisante pour conférer à ladite phase huileuse une viscosité supérieure ou égale à 750 Pa.s au cisaillement de 1 $s^{-1}$, à 25 °C.

4. Particules selon l'une quelconque des revendications précédentes, **caractérisées en ce qu'**elles possèdent une taille moyenne inférieure ou égale à 12 µm, et plus particulièrement inférieure ou égale à 11 µm.

5. Particules selon la revendication précédente, **caractérisées en ce qu'**elles possèdent une taille moyenne variant de 100 nm à 5 µm, voire allant de 100 nm à 12 µm, et en particulier allant de 150 nm à 11 µm.

6. Particules selon l'une quelconque des revendications précédentes, **caractérisées en ce que** la phase huileuse structurée possède un point de fusion inférieur ou égal à 95 °C, de préférence variant de 50 à 90 °C.

7. Particules selon l'une quelconque des revendications précédentes, **caractérisées en ce que**, lorsqu'elles ont une

taille moyenne micrométrique, elles possèdent un coefficient d'uniformité inférieur ou égal à 0,45.

8. Particules selon l'une quelconque des revendications 1 à 7, **caractérisées en ce que**, lorsqu'elles ont une taille moyenne submicrométrique, elles possèdent un indice de polydispersité inférieur ou égal à 0,35.

9. Particules selon l'une quelconque des revendications précédentes, **caractérisées en ce que** leur forme est substantiellement sphérique.

10. Particules selon l'une quelconque des revendications précédentes, **caractérisées en ce que** la phase huileuse comprend au moins une huile non volatile.

11. Particules selon l'une quelconque des revendications précédentes, **caractérisées en ce que** la phase huileuse est présente en une teneur variant de 10 à 99 % en poids, en particulier de 20 à 99 % en poids, plus particulièrement de 30 à 99 % en poids par rapport au poids total de la particule.

12. Particules selon l'une quelconque des revendications précédentes, **caractérisées en ce que** la phase huileuse comprend au moins une huile choisie parmi une huile végétale, une huile animale, une huile synthétique, une huile minérale et leurs mélanges.

13. Particules selon la revendication précédente, **caractérisées en ce que** la phase huileuse comprend au moins une huile est choisie parmi le squalane, l'isononanoate d'isononyle, le lauroylsarcosinate d'isopropyle, l'octyldodecanol, et leurs mélanges.

14. Particules selon l'une quelconque des revendications précédentes, **caractérisées en ce que** le polymère gélifiant est choisi parmi l'éthylènediamine/stéaryle dimerdilinoléate, le polymère semi-cristallin de type poly-$C_{10-30}$ alkylacrylate, et leurs mélanges.

15. Particules selon l'une quelconque des revendications précédentes, **caractérisées en ce que** le polymère gélifiant est présent en une teneur variant de 1 à 80 % en poids, voire de 1 à 60 % en poids par rapport au poids total de la particule.

16. Particules selon l'une quelconque des revendications précédentes, **caractérisées en ce que** le rapport pondéral entre le polymère gélifiant et la phase huileuse de la particule varie de 0,01 à 4, voire de 0,05 à 2, et en particulier de 0,1 à 1.

17. Particules selon l'une quelconque des revendications précédentes, **caractérisées en ce qu'**elles comprennent en outre au moins une substance active.

18. Particules selon la revendication précédente, **caractérisées en ce que** la substance active est choisie parmi un agent antibactérien, un agent antifongique, un agent séborégulateur, un agent sébostimulateur, un agent kératolytique, un agent de traitement de l'acné, un antibiotique de structure macrolidique, un agent inhibiteur de la chute du cheveu, un agent inhibiteur de la pousse du cheveu, un agent antipelliculaire, un antioxydant, un agent astringent, un agent réducteur de pores, un agent anti-transpirant, une vitamine, un agent anti-inflammatoire, et leurs mélanges.

19. Particules selon la revendication 17 ou 18, **caractérisées en ce que** la substance active est présente en une teneur variant de 0,05 à 70 % en poids, de 0,1 à 50 % en poids, et en particulier de 0,5 à 40 % en poids par rapport au poids total de la particule.

20. Dispersion en phase aqueuse et/ou hydrosoluble comprenant des particules selon l'une quelconque des revendications 1 à 19.

21. Dispersion selon la revendication précédente, **caractérisée en ce qu'**elle comprend en outre au moins un agent tensioactif choisi parmi un agent tensioactif ionique, un agent tensioactif non ionique, et leurs mélanges.

22. Dispersion selon l'une quelconque des revendications 20 ou 21, **caractérisée en ce qu'**elle comprend en outre au moins un polymère émulsionnant hydrophobe.

23. Dispersion selon l'une quelconque des revendications 20 à 22, **caractérisée en ce qu'**elle comprend en outre au

moins un polymère hydrophile gélifiant.

**24.** Dispersion selon l'une quelconque des revendications 20 à 23, **caractérisée en ce que** les particules d'huiles structurées dispersées dans la phase aqueuse représentent une fraction volumique huileuse variant de 5 à 89 % en poids par rapport au poids total de la dispersion, de préférence de 20 à 85 % en poids, voire de 40 à 80 % en poids, et en particulier de 60 à 80 % en poids.

**25.** Composition cosmétique ou dermatologique comprenant au moins des particules telles que définies selon l'une quelconque des revendications 1 à 19, et/ou au moins une dispersion telle que définie selon l'une quelconque des revendications 20 à 24.

**26.** Procédé de fabrication d'une dispersion de particules huileuses calibrées et sphériques comprenant au moins une phase huileuse structurée par au moins un polymère gélifiant et possédant une taille moyenne inférieure ou égale à 15 $\mu$m, comprenant au moins les étapes consistant à :

- émulsifier un mélange d'au moins une huile ou phase huileuse et d'au moins un polymère gélifiant de ladite phase huileuse avec une phase aqueuse et/ou hydrosoluble, à une température supérieure à la température de gélification dudit polymère,
- soumettre le mélange à un processus conduisant à obtenir lesdites particules huileuses, à une température d'au moins 5 à 10 °C supérieure à la température de fusion du mélange mis en oeuvre dans l'étape précédente, et
- refroidir la dispersion des particules ainsi obtenues.

**27.** Procédé selon la revendication précédente, **caractérisé en ce que** ledit polymère gélifiant est tel que défini en revendications 2 et 15 à 16.

**28.** Procédé selon la revendication 26 ou 27, **caractérisé en ce que** ledit processus conduisant à obtenir des particules est adapté à l'obtention de particules d'une taille moyenne allant d'environ 1 à environ 15 micromètres.

**29.** Procédé selon la revendication précédente, **caractérisé en ce que** ledit processus est choisi parmi le cisaillement contrôlé d'émulsions viscoélastiques, un procédé utilisant un moulin colloïdal, un procédé utilisant un mélangeur statique, un procédé utilisant un micro mélangeur, un procédé utilisant une pale cadre, un procédé utilisant un extrudeur-malaxeur, un procédé utilisant une membrane poreuse, un procédé utilisant un contrôle du mûrissement, un procédé utilisant un gonflement d'un latex "templating agent", un procédé utilisant les instabilités de Rayleigh, et un procédé utilisant un fractionnement d'émulsion polydisperse.

**30.** Procédé selon la revendication 26 ou 27, **caractérisé en ce que** le processus conduisant à obtenir des particules est adapté à l'obtention de particules d'une taille moyenne allant d'environ 150 nm à environ 1 $\mu$m.

**31.** Procédé selon la revendication précédente, **caractérisé en ce que** ledit processus est choisi parmi un procédé développant un cisaillement et un procédé mettant en jeu une inversion de phase.

**32.** Procédé selon l'une quelconque des revendications 26 à 31, **caractérisé en ce que** la température de l'étape d'émulsification est supérieure à 40 °C.

**33.** Procédé selon l'une quelconque des revendications 26 à 32, **caractérisé en ce que** ledit mélange comprend en outre une substance active.

**34.** Particules ou dispersion de particules selon l'une quelconque des revendications 1 à 24 susceptibles d'être obtenues par un procédé tel que défini selon l'une quelconque des revendications 26 à 33.

**35.** Utilisation de particules selon l'une quelconque des revendications 1 à 19 et 34 et/ou d'au moins une dispersion selon l'une quelconque des revendications 20 à 24 et 34 dans une composition cosmétique destinée au traitement des peaux grasses.

**36.** Utilisation de particules selon l'une quelconque des revendications 1 à 19 et 34 et/ou d'au moins une dispersion selon l'une quelconque des revendications 20 à 24 et 34 pour la préparation d'une composition dermatologique destinée au traitement de l'acné.

**37.** Procédé de maquillage et/ou de soin non thérapeutique de la peau, comprenant au moins une étape d'application sur celle-ci d'au moins une composition comprenant des particules telles que définies selon l'une quelconque des revendications 1 à 19 et 34 et/ou d'au moins une dispersion telle que définie selon l'une quelconque des revendications 20 à 24 et 34.

**Claims**

**1.** Calibrated and spherical oily particles comprising at least one oily phase structured with at least one gelling polymer, **characterized in that** they have a mean size of less than or equal to 15 μm and **in that** the said structured oily phase has a melting point of greater than or equal to 40°C, and **in that** their circularity index is between 0.9 and 1.

**2.** Calibrated and spherical oily particles according to claim 1, **characterized in that** the gelling polymer is chosen from a semi-crystalline polymer, a polyamide, a silicone polyamide, a polysaccharide monoalkyl ester or polyalkyl ester, a diblock and/or triblock and/or multiblock and/or radial-block copolymer, and mixtures thereof.

**3.** Calibrated and spherical oily particles according to claim 1, **characterized in that** the said gelling polymer is of a nature and/or in an amount sufficient to give the said oily phase a viscosity of greater than or equal to 750 Pa.s at a shear of 1 $^{s-1}$, a$^{t}$ 25°C.

**4.** Particles according to any one of the preceding claims, **characterized in that** they have a mean size of less than or equal to 12 μm and more particularly less than or equal to 11 μm.

**5.** Particles according to the preceding claim, **characterized in that** they have a mean size ranging from 100 nm to 5 μm, or even ranging from 100 nm to 12 μm, and in particular ranging from 150 μm to 11 μm.

**6.** Particles according to any one of the preceding claims, **characterized in that** the structured oily phase has a melting point of less than or equal to 95°C, preferably ranging from 50 to 90°C.

**7.** Particles according to any one of the preceding claims, **characterized in that**, when they have a mean micrometric size, they have a coefficient of uniformity of less than or equal to 0.45.

**8.** Particles according to any one of Claims 1 to 7, **characterized in that**, when they have a mean submicrometric size, they have a polydispersity index of less than or equal to 0.35.

**9.** Particles according to any one of the preceding claims, **characterized in that** they are of substantially spherical form.

**10.** Particles according to any one of the preceding claims, **characterized in that** the oily phase comprises at least one non-volatile oil.

**11.** Particles according to any one of the preceding claims, **characterized in that** the oily phase is present in an amount ranging from 10% to 99% by weight, in particular from 20% to 99% by weight and more particularly from 30% to 99% by weight relative to the total weight of the particle.

**12.** Particles according to any one of the preceding claims, **characterized in that** the oily phase comprises at least one oil chosen from a plant oil, an animal oil, a synthetic oil and a mineral oil, and mixtures thereof.

**13.** Particles according to the preceding claim, **characterized in that** the oily phase comprises at least one oil chosen from squalane, isononyl isononanoate, isopropyl lauroylsarcosinate and octyldodecanol, and mixtures thereof.

**14.** Particles according to any one of the preceding claims, **characterized in that** the gelling polymer is chosen from ethylenediamine/stearyl dimerdilinoleate and the semi-crystalline polymer of poly-$C_{10-30}$ alkyl acrylate type, and mixtures thereof.

**15.** Particles according to any one of the preceding claims, **characterized in that** the gelling polymer is present in an amount ranging from 1% to 80% by weight or even from 1% to 60% by weight relative to the total weight of the particle.

**16.** Particles according to any one of the preceding claims, **characterized in that** the weight ratio between the gelling

polymer and the oily phase of the particle ranges from 0.01 to 4 or even from 0.05 to 2, and in particular from 0.1 to 1.

**17.** Particles according to any one of the preceding claims, **characterized in that** they also comprise at least one active substance.

**18.** Particles according to the preceding claim, **characterized in that** the active substance is chosen from an antibacterial agent, an antifungal agent, a sebo-regulatory agent, a sebo-stimulatory agent, a keratolytic agent, an agent for treating acne, an antibiotic of macrolide structure, an agent for inhibiting hair loss, an agent for inhibiting hair growth, an antidandruff agent, an antioxidant, an astringent, a pore-reducing agent, an antiperspirant, a vitamin and an anti-inflammatory agent, and mixtures thereof.

**19.** Particles according to Claim 17 or 18, **characterized in that** the active substance is present in a content ranging from 0.05% to 70% by weight, from 0.1% to 50% by weight and in particular from 0.5% to 40% by weight relative to the total weight of the particle.

**20.** Aqueous and/or water-soluble phase dispersion comprising particles according to any one of Claims 1 to 19.

**21.** Dispersion according to the preceding claim, **characterized in that** it also comprises at least one surfactant chosen from an ionic surfactant and a nonionic surfactant, and mixtures thereof.

**22.** Dispersion according to either of Claims 20 and 21, **characterized in that** it also comprises at least one hydrophobic emulsifying polymer.

**23.** Dispersion according to any one of Claims 20 to 22, **characterized in that** it also comprises at least one hydrophilic gelling polymer.

**24.** Dispersion according to any one of Claims 20 to 23, **characterized in that** the structured oil particles dispersed in the aqueous phase represent an oily volume fraction ranging from 5% to 89% by weight, preferably from 20% to 85% by weight or even from 40% to 80% by weight, and in particular from 60% to 80% by weight, relative to the total weight of the dispersion.

**25.** Cosmetic or dermatological composition comprising at least some particles as defined according to any one of Claims 1 to 19, and/or at least one dispersion as defined according to any one of Claims 20 to 24.

**26.** Process for manufacturing a dispersion of calibrated and spherical oily particles comprising at least one oily phase structured with at least one gelling polymer and having a mean size of less than or equal to 15 μm, comprising at least the steps consisting in:

- emulsifying a mixture of at least one oil or an oily phase and at least one oily-phase-gelling polymer with an aqueous and/or water-soluble phase at a temperature above the gel point of the polymer,
- subjecting the mixture to a process leading to the production of the said oily particles, at a temperature at least 5 to 10°C above the melting point of the mixture used in the preceding step, and
- cooling the particle dispersion thus obtained.

**27.** Process according to the preceding claim, **characterized in that** the said gelling polymer is as defined in Claims 2 and 15 to 16.

**28.** Process according to Claim 26 or 27, **characterized in that** the said process leading to the production of particles is suitable for obtaining particles with a mean size ranging from about 1 to about 15 micrometres.

**29.** Process according to the preceding claim, **characterized in that** the said process is chosen from the controlled shear of viscoelastic emulsions, a process using a colloidal mill, a process using a static mixer, a process using a micromixer, a process using a frame paddle, a process using an extruder-blender, a process using a porous membrane, a process using maturation control, a process using swelling of a "templating agent" latex, a process using Rayleigh instabilities, and a process using fractionation of a polydisperse emulsion.

**30.** Process according to Claim 26 or 27, **characterized in that** the process leading to the production of particles is suitable for obtaining particles with a mean size ranging from about 150 nm to about 1 μm.

31. Process according to the preceding claim, **characterized in that** the said process is chosen from a process developing a shear and a process involving a phase inversion.

32. Process according to any one of Claims 26 to 31, **characterized in that** the temperature of the emulsification step is greater than 40°C.

33. Process according to any one of Claims 26 to 32, **characterized in that** the said mixture also comprises an active substance.

34. Particles or particle dispersion according to any one of Claims 1 to 24, which may be obtained according to the process as defined according to any one of Claims 26 to 33.

35. Use of particles according to any one of Claims 1 to 19 and 34 and/or of at least one dispersion according to any one of Claims 20 to 24 and 34 in a cosmetic composition for treating greasy skin.

36. Use of particles according to any one of Claims 1 to 19 and 34 and/or of at least one dispersion according to any one of Claims 20 to 24 and 34 for the preparation of a dermatological composition for treating acne.

37. Non-therapeutic process for making up and/or caring for the skin, comprising at least one step of applying thereto at least one composition comprising particles as defined according to any one of Claims 1 to 19 and 34 and/or at least one dispersion as defined according to any one of Claims 20 to 24 and 34.

## Patentansprüche

1. Sortierte kugelförmige Ölpartikel, die mindestens eine durch mindestens ein gelbildendes Polymer strukturierte Ölphase umfassen, **dadurch gekennzeichnet, dass** sie eine mittlere Größe von kleiner oder gleich 15 μm besitzen, dass die strukturierte Ölphase einen Schmelzpunkt von größer oder gleich 40 °C besitzt, und dadurch, dass ihr Kreisförmigkeitsindex zwischen 0,9 und 1, Grenzen eingeschlossen, liegt.

2. Sortierte kugelförmige Ölpartikel nach Anspruch 1, **dadurch gekennzeichnet, dass** das gelbildende Polymer aus einem semikristallinen Polymer, einem Polyamid, einem silikonierten Polyamid, einem Polysaccharidmonoalkylester oder -polyalkylester, einem Zweiblock- und/oder Dreiblock und/oder Mehrblockcopolymer und/oder aus einem radialen Blockcopolymer und ihren Gemischen ausgewählt ist.

3. Sortierte kugelförmige Ölpartikel nach Anspruch 1, **dadurch gekennzeichnet, dass** das gelbildende Polymer so ist und/oder in einem Gehalt vorhanden ist, der ausreicht, um der Ölphase eine Viskosität von größer oder gleich 750 Pa.s bei einer Scherung von 1 $s^{-1}$ bei 25 °C zu verleihen.

4. Partikel nach einem der vorhergehenden Ansprüche, **dadurch gekennzeichnet, dass** sie eine mittlere Größe von kleiner oder gleich 12 μm und spezieller von kleiner oder gleich 11 μm besitzen.

5. Partikel nach dem vorhergehenden Anspruch, **dadurch gekennzeichnet, dass** sie eine mittlere Größe besitzen, die von 100 nm bis 5 μm, sogar von 100 nm bis 12 μm und insbesondere von 150 nm bis 11 μm variiert.

6. Partikel nach einem der vorhergehenden Ansprüche, **dadurch gekennzeichnet, dass** die strukturierte Ölphase einen Schmelzpunkt von kleiner oder gleich 95 °C, der vorzugsweise von 50 bis 90 °C variiert, aufweist.

7. Partikel nach einem der vorhergehenden Ansprüche, **dadurch gekennzeichnet, dass**, wenn sie eine mittlere Größe im Mikrometerbereich besitzen, sie einen Gleichmäßigkeitskoeffzienten von kleiner oder gleich 0,45 aufweisen.

8. Partikel nach einem der Ansprüche 1 bis 7, **dadurch gekennzeichnet, dass** wenn sie eine mittlere Größe im Submikrometerbereich besitzen, sie einen Polydispersitätsindex von kleiner oder gleich 0,35 aufweisen.

9. Partikel nach einem der vorhergehenden Ansprüche, **dadurch gekennzeichnet, dass** ihre Form im Wesentlichen kugelförmig ist.

10. Partikel nach einem der vorhergehenden Ansprüche, **dadurch gekennzeichnet, dass** die Ölphase mindestens ein nichtflüchtiges Öl umfasst.

11. Partikel nach einem der vorhergehenden Ansprüche, **dadurch gekennzeichnet, dass** die Ölphase in einem Gehalt vorhanden ist, der von 10 bis 99 Gew.-%, insbesondere von 20 bis 99 Gew.-%, spezieller von 30 bis 99 Gew.-%, bezogen auf das Gesamtgewicht des Partikels, variiert.

12. Partikel nach einem der vorhergehenden Ansprüche, **dadurch gekennzeichnet, dass** die Ölphase mindestens ein Öl umfasst, das aus einen pflanzlichen Öl, einem tierischen Öl, einem synthetischen Öl, einem mineralischen Öl und ihren Gemischen ausgewählt ist.

13. Partikel nach dem vorhergehenden Anspruch, **dadurch gekennzeichnet, dass** die Ölphase mindestens ein Öl umfasst, das aus Squalan, Isononylisononanoat, Isopropyllauroylsarcosinat, Octyldodecanol und ihren Gemischen ausgewählt ist.

14. Partikel nach einem der vorhergehenden Ansprüche, **dadurch gekennzeichnet, dass** das gelbildende Polymer aus Ethylendiamin/Stearyldimerdilinoleat, semikristallinem Poly-$C_{10-30}$-Alkylacrylat-Polymer und ihren Gemischen ausgewählt ist.

15. Partikel nach einem der vorhergehenden Ansprüche, **dadurch gekennzeichnet, dass** das gelbildende Polymer in einem Gehalt vorhanden ist, der von 1 bis 80 Gew.-%, sogar von 1 bis 60 Gew.-%, bezogen auf das Gesamtgewicht des Partikels, variiert.

16. Partikel nach einem der vorhergehenden Ansprüche, **dadurch gekennzeichnet, dass** das Gewichtsverhältnis zwischen dem gelbildenden. Polymer und der Ölphase des Partikels von 0,01 bis 4, sogar von 0,05 bis 2 und insbesondere von 0,1 bis 1 variiert.

17. Partikel nach einem der vorhergehenden Ansprüche, **dadurch gekennzeichnet, dass** sie weiterhin mindestens einen Wirkstoff umfassen.

18. Partikel nach dem vorhergehenden Anspruch, **dadurch gekennzeichnet, dass** der Wirkstoff ausgewählt ist aus einem antibakteriellen Mittel, einem antifungalen Mittel, einem seboregulierenden Mittel, einem sebostimulierenden Mittel, einem keratolytischen Mittel, einem Akne-Behandlungsmittel, einem Antibiotikum mit Makrolid-Struktur, einem den Haarausfall hemmenden Mittel, einem den Haarwuchs hemmenden Mittel, einem Antischuppenmittel, einem Antioxidans, einem adstringierenden Mittel, einem Mittel zur Porenreduktion, einem Antitranspirant, einem Vitamin, einem entzündungshemmenden Mittel und ihren Gemischen.

19. Partikel nach Anspruch 17 oder 18, **dadurch gekennzeichnet, dass** der Wirkstoff in einem Gehalt vorhanden ist, der von 0,05 bis 70 Gew.-%, von 0,1 bis 50 Gew.-% und insbesondere von 0,5 bis 40 Gew.-%, bezogen auf das Gesamtgewicht des Partikels, variiert.

20. Dispersion in wässriger oder wasserlöslicher Phase, umfassend Partikel nach einem der Ansprüche 1 bis 19.

21. Dispersion nach dem vorhergehenden Anspruch, **dadurch gekennzeichnet, dass** sie weiterhin mindestens ein grenzflächenaktives Mittel umfasst, ausgewählt aus einem ionischen grenzflächenaktiven Mittel, einem nicht ionischen grenzflächenaktiven Mittel und ihren Gemischen.

22. Dispersion nach einem der Ansprüche 20 oder 21, **dadurch gekennzeichnet, dass** sie weiterhin mindestens ein emulgierendes hydrophobes Polymer umfasst.

23. Dispersion nach einem der Ansprüche 20 bis 22, **dadurch gekennzeichnet, dass** sie weiterhin mindestens ein hydrophiles gelbildendes Polymer umfasst.

24. Dispersion nach einem der Ansprüche 20 bis 23, **dadurch gekennzeichnet, dass** die strukturierten in der wässrigen Phase dispergierten Partikel von Ölen eine ölige Volumenfraktion darstellen, die von 5 bis 89 Gew.-%, bezogen auf das Gesamtgewicht der Dispersion, vorzugsweise von 20 bis 85 Gew.-%, sogar von 40 bis 80 Gew.-% und insbesondere von 60 bis 80 Gew.-%,, variiert.

**25.** Kosmetische oder dermatologische Zusammensetzung, umfassend mindestens Partikel, wie nach einem der Ansprüche 1 bis 19 definiert, und/oder mindestens eine Dispersion, wie nach einem der Ansprüche 20 bis 24 definiert.

**26.** Verfahren zur Herstellung einer Dispersion von sortierten kugelförmigen öligen Partikeln, die mindestens eine durch mindestens ein gelbildendes Polymer strukturierte Ölphase umfassen und eine mittlere Größe von kleiner oder gleich 15 μm aufweisen, umfassend mindestens die Schritte, bestehend aus:

- Emulgieren eines Gemisches von mindestens einem Öl oder einer Ölphase und von mindestens einem gelbildenden Polymer der Ölphase mit einer wässrigen und/oder wasserlöslichen Phase bei einer Temperatur oberhalb der Gelbildungstemperatur des Polymers,
- Durchführen mit dem Gemisch eines Verfahrens, das zum Erhalt der ölhaltigen Partikel führt, bei einer Temperatur von mindestens 5 bis 10 °C über der Schmelztemperatur des im vorhergehenden Schritt eingesetzten Gemisches, und
- Abkühlen der Dispersion der so erhaltenen Partikel.

**27.** Verfahren nach dem vorhergehenden Anspruch, **dadurch gekennzeichnet, dass** das gelbildende Polymer so ist wie in den Ansprüchen 2 und 15 bis 16 definiert.

**28.** Verfahren nach Anspruch 26 oder 27, **dadurch gekennzeichnet, dass** das Verfahren, das zum Erhalt der Partikel führt, dazu ausgelegt ist, Partikel einer mittleren Größe zu erhalten, die von etwa 1 bis etwa 15 μm reicht.

**29.** Verfahren nach dem vorhergehenden Anspruch, **dadurch gekennzeichnet, dass** das Verfahren ausgewählt ist aus der kontrollierten Scherung von viskoelastischen Emulsionen, einem Verfahren unter Verwendung von einer Kolloidmühle, einem Verfahren unter Verwendung eines statischen Mischers, einem Verfahren unter Verwendung eines Mikromischers, einem Verfahren unter Verwendung einer Impellermühle, einem Verfahren unter Verwendung eines Extruder-Mischers, einem Verfahren unter Verwendung einer porösen Membran, einem Verfahren unter Verwendung einer Reifekontrolle, einem Verfahren unter Verwendung eines Aufblasens eines Latex-"templating agent", einem Verfahren unter Verwendung der Rayleigh-Instabilitäten und einem Verfahren unter Verwendung einer polydisersen Emulsionsfraktionierung.

**30.** Verfahren nach Anspruch 26 oder 27, **dadurch gekennzeichnet, dass** das Verfahren, das zum Erhalt der Partikeln führt, dazu ausgelegt ist, Partikel einer mittleren Größe von etwa 150 nm bis 1 μm zu erhalten.

**31.** Verfahren nach dem vorhergehenden Anspruch, **dadurch gekennzeichnet, dass** das Verfahren aus einem Verfahren ausgewählt ist, das eine Scherung entwickelt, und einem Verfahren, das eine Phaseninversion einsetzt.

**32.** Verfahren nach einem der Ansprüche 26 bis 31, **dadurch gekennzeichnet, dass** die Temperatur des Emulgierschrittes größer ist als 40 °C.

**33.** Verfahren nach einem der Ansprüche 26 bis 32, **dadurch gekennzeichnet, dass** das Gemisch weiterhin einen Wirkstoff umfasst.

**34.** Partikel oder Dispersion von Partikeln nach einem der Ansprüche 1 bis 24, die sich durch ein Verfahren wie nach einem der Ansprüche 26 bis 33 definiert erhalten lassen.

**35.** Verwendung von Partikeln nach einem der Ansprüche 1 bis 19 und 34 und/oder von mindestens einer Dispersion nach einem der Ansprüche 20 bis 24 und 34 in einer kosmetischen Zusammensetzung, die zur Behandlung von fettiger Haut bestimmt ist.

**36.** Verwendung von Partikeln nach einem der Ansprüche 1 bis 19 und 34 und/oder von mindestens einer Dispersion nach einem der Ansprüche 20 bis 24 und 34 zur Herstellung einer dermatologischen Zusammensetzung, die zur Behandlung von Akne bestimmt ist.

**37.** Nicht therapeutisches Schmink- und/oder Pflegeverfahren der Haut, umfassend mindestens einen Schritt des Aufbringens auf diese von mindestens einer Zusammensetzung, die Partikel, wie nach einem der Ansprüche 1 bis 19 und 34 definiert, umfasst, und/oder von mindestens einer Dispersion, wie nach einem der Ansprüche 20 bis 24 und 34 definiert.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4690825 A
- WO 9953904 A
- US 6737394 B
- WO 02092043 A
- EP 0375520 A
- FR 0302809
- EP 951897 A
- US 5156911 A
- WO 0119333 A
- US 5736125 A
- US 5519063 A
- EP 0550745 A
- US 5783657 A
- US 3645705 A
- US 3148125 A
- US 5500209 A
- US 5756082 A
- EP 0497144 A
- WO 9842298 A
- US 6225390 B
- US 6160054 A
- US 6174968 B
- US 5468477 A
- US 5725882 A
- EP 0852949 A
- WO 9318743 A
- US 6407056 B
- US 6171595 B
- US 6075052 A
- US 6020006 A
- US 4885289 A
- US 4720489 A
- US 5132293 A
- US 5096911 A
- US 5095007 A
- US 5143925 A
- US 5328686 A
- US 5440090 A
- US 5364885 A
- US 5411991 A
- US 5648394 A
- US 5468476 A
- US 5475763 A
- US 5455608 A
- US 5674477 A
- US 5728736 A
- US 5652273 A
- WO 9427586 A
- WO 9427563 A
- WO 9803149 A
- US 6375948 B
- US 6541018 B
- US 6335022 B
- US 6375960 B
- US 6689371 B
- US 6461625 B
- US 6413527 B
- US 6274150 B
- US 6464990 B
- US 6120778 A
- EP 1466588 A
- US 20030138465 A
- US 20040214913 A
- US 20030147832 A
- US 20020198328 A
- FR 2856923
- EP 0447318 A
- FR 2747321
- US 5558820 A
- US 5688842 A
- WO 0240574 A
- US 5326484 A
- US 6160061 A
- EP 719087 A


### Littérature non-brevet citée dans la description


- **S. NOJIMA.** Melting behavior of poly(Σ -caprolactone)-block-polybutadiène copolymers. *Macromolécules,* 1999, vol. 32, 3727-3734
- **B. BOUTEVIN et al.** Study of morphological and mechanical properties of PP/PBT. *Polymer Bulletin,* 1995, vol. 34, 117-123
- **P. RANGARAJAN et al.** Morphology of semi-crystalline block copolymers of ethylene- (ethylene-alt-propylene. *Macromolecules,* 1993, vol. 26, 4640-4645
- **P. RICHTER et al.** Polymer agregates with crystalline cores : the system poly(ethylene)- poly(ethylene-propylene. *Macromolécules,* 1997, vol. 30 (25), 1053-1068

- **I.W. HAMLEY.** Cristallization in block copolymers. *Advances in Polymer Science,* 1999, vol. 148, 113-137
- **WEITZ.** *Langmuir,* 2000, vol. 16, 347-351
- **BIBETTE.** *J. Coll. Int. Sci.,* 1991, vol. 147 (2), 474-478